# FASCICULE DE BREVET EUROPEEN

(11) **EP 2 882 415 B1**
(45) Date de publication et mention de la délivrance du brevet: **18.03.2020**
(21) Numéro de dépôt: 13773275.6
(22) Date de dépôt: 13.08.2013
(51) Int. Cl.: A61K 8/73, A61Q 19/00, A61K 31/719, A61K 31/728, A61K 31/734

(54) **INGREDIENT HYDRATANT COSMETIQUE OU PHARMACEUTIQUE**
FEUCHTIGKEITSSPENDENDER BESTANDTEIL EINES KOSMETIKUMS ODER PHARMAZEUTIKUMS
COSMETIC OR PHARMACEUTICAL MOISTURISING INGREDIENT

(30) Priorité: 13.08.2012 FR 1257786; 20.08.2012 US 201261684983 P
(43) Date de publication de la demande: 17.06.2015
(73) Titulaire: BASF Beauty Care Solutions France S.A.S., 69007 Lyon (FR)
(72) Inventeur: BONNET, Isabelle, F-69007 Lyon (FR); VERRECCHIA, Nicolas, F-69680 Chassieu (FR)
(74) Mandataire: Cabinet Beau de Loménie
(86) Numéro de dépôt international: PCT/FR2013/051933
(87) Numéro de publication internationale: WO 2014/027163

(56) Documents cités:
- WO-A1-2005/095462
- JP-A- S57 185 208
- US-A1- 2009 041 814
- DATABASE WPI Week 201146 6 juin 2013 (2013-06-06) Thomson Scientific, London, GB; AN 2011-G64149 XP002698376, -& CN 102 048 712 A (ZHEJIANG WOLWO BIOTECH CO LTD) 11 mai 2011 (2011-05-11)
- DATABASE GNPD [Online] MINTEL; 31 décembre 2009 (2009-12-31), "Face Up Serum", XP002698377, Database accession no. 1226038
- DATABASE GNPD [Online] MINTEL; 29 février 2008 (2008-02-29), "Serum", XP002698378, Database accession no. 867707
- DATABASE GNPD [Online] MINTEL; 31 mai 2011 (2011-05-31), "Eye Gel Cream", XP002698379, Database accession no. 1542203
- "Moisturizers in the skin care", Beauty Forum, 1 January 2011 (2011-01-01), pages 86-88, XP055035676, Retrieved from the Internet: URL:http://www.dermaviduals.com/cms/upload /Publikationen_english/BF-03-11-Moisturize r-engl.pdf [retrieved on 2012-08-16]
- SYLVIE VERDIER-SEVRAIN MD ET AL: "SKIN HYDRATION: A REVIEW ON ITS MOLECULAR MECHANISMS", JOURNAL OF COSMETIC DERMATOLOGY, BLACKWELL SCIENCE, OXFORD, GB, vol. 6, no. 2, 1 January 2007 (2007-01-01) , pages 75-82, XP001536242, ISSN: 1473-2130, DOI: 10.1111/J.1473-2165.2007.00300.X

## Description

La présente invention concerne le domaine de la cosmétique et le domaine pharmaceutique, en particulier dermatologique. La présente invention concerne tout particulièrement un nouveau mélange d'ingrédients permettant de diminuer les pertes insensibles en eau et ainsi maintenir l'hydratation de la peau.

La peau est un organe en contact constant avec l'extérieur. Elle joue donc un rôle d'échange mais aussi et surtout de barrière vis-à-vis de l'extérieur.
La couche superficielle de la peau, appelée couche cornée, constitue en effet le premier rempart protégeant la peau, sous la forme d'une fine pellicule. Celle -ci par sa nature hydrophobique constitue un rempart à la diffusion de l'eau, principal constituant des cellules et évitant ainsi la déshydratation. Néanmoins, il existe toujours des pertes d'eau dites transépidermales ou autrement dénommées pertes insensibles en eau. Si la couche cornée est affaiblie, en particulier par des agressions ou dans des situations pathologiques, la barrière cutanée est rompue et ces pertes insensibles en eau augmentent. La peau devient sèche et plus vulnérable aux agressions extérieures. Si cette déshydratation cutanée se poursuit, elle peut induire de réelles pathologies. Le maintien de la teneur en eau via le rôle de la barrière cutanée est donc essentiel pour le maintien des fonctionnalités et l'aspect esthétique de la peau.
L'apport d'actifs hydratants et la limitation des pertes insensibles en eau sont les deux axes principaux pour maintenir et renforcer cette barrière cutanée et par conséquent le bon état général de la peau. De nouveaux actifs hydratants et/ou capables de diminuer les pertes insensibles en eau présentent donc ainsi un grand intérêt au niveau cosmétique et pharmaceutique, notamment dermatologique.

À ce jour il existe de nombreux actifs hydratants tels que ceux décrits dans les demandes de brevet WO2005/095462 et FR2902333. Toutefois, les compositions existantes actuellement sur le marché n'ont qu'un pouvoir hydratant de courte durée. Il existe donc le besoin de pouvoir maintenir l'hydratation de la peau sur une longue durée c'est-à-dire pendant au moins 10 heures, voir 24 heures.

Le pullulan est un polysaccharide naturel (polymère d'ose) constitué d'unités de maltotriose (un triholoside de glucose), aussi connu comme 1'α-1,4- ;α-1,6-glucane. Les trois unités de glucose qui composent le maltotriose sont reliées par une liaison osidique du type α-1,4, tandis que les maltotrioses sont connectés entre eux par des liaisons osidiques du type α-1,61. Les applications du pullulan sont multiples.
Il est surtout connu comme étant un agent épaississant ou filmogène comme décrit dans la demande de brevet US 2003/0082221. Il est également connu pour son aptitude à capter et retenir l'eau comme décrit dans la demande de brevet FR7434619.
L'acide hyaluronique, ses sels et dérivés font partis des actifs hydratants les plus connus et les plus utilisés à l'heure actuelle. L'acide hyaluronique est un polymère de disaccharides, eux-mêmes composés d'acide D-glucuronique et de D-N-acétylglucosamine, liés entre eux par des liaisons glycosidiques alternées beta-1,4 et beta-1,3. Il s'agit donc d'un glycosaminoglycane naturel. C'est l'un des principaux composants de la matrice extracellulaire. Ses différentes fractions, en fonction de leurs poids moléculaires sont utilisables en tant qu'agent hydratant comme décrit dans la demande de brevet US2008/0003271.
La stabilisation de l'acide hyaluronique a été investiguée dans la demande de brevet US 2009/0042834 qui décrit l'association d'une composition de glycosaminoglycane tels que l'acide hyaluronique avec un agent stabilisant choisi parmi les polysaccharides à longue chaîne hydroxyle polyanioniques tels que l'alginate de sodium et l'acide alginique. Cette stabilisation a pour objectif de palier la perte de viscosité observée avec le temps dans les formulations d'acide hyaluronique. De telles compositions d'acide hyaluronique ainsi stabilisées sont utilisées pour les usages habituels de l'acide hyaluronique, notamment ses propriétés hydratantes. Aucune amélioration des propriétés de l'acide hyaluronique n'est toutefois décrite, à l'exception d'un meilleur maintien des propriétés de celui-ci en formulation. En outre, il n'est pas suggéré d'ajouter du pullulan à une telle composition afin d'en améliorer l'effet. Par ailleurs, et comme cela est mis en évidence par les inventeurs dans l'exemple 3, la stabilisation de l'acide hyaluronique par l'acide alginique n'améliore pas son efficacité sur les pertes insensibles en eau.

L'acide alginique et ses dérivés (base conjuguée, sels et esters) sont des polysaccharides naturels obtenus à partir d'une famille d'algues brunes : les laminaires ou les fucus. L'alginate est un polymère formé de deux monomères liés ensemble : le mannuronate ou acide mannuronique dont certains sont acétylés et le guluronate ou acide guluronique. La liaison se fait via beta 1-4. Les alginates sont utilisés comme épaississants, gélifiants, émulsifiants et stabilisants de produits industriels les plus variés. Ils sont également connus comme ayant des propriétés de rétention d'eau.
Toutefois il n'a jamais été suggéré de l'associer au pullulan pour obtenir un produit ayant des pouvoirs d'hydratation élevés.

Or, de façon surprenante, les inventeurs ont découvert qu'il existait une synergie sur la diminution des pertes insensibles en eau entre le pullulan et un mélange de polysaccharides consistant en l'acide hyaluronique et l'acide alginique, éventuellement sous la forme de leurs sels ou dérivés respectifs. Cette association, après application sur la peau et/ou les muqueuses, permet d'en réduire les pertes insensibles en eau, et ainsi de maintenir la teneur en eau de la peau et/ou des muqueuses. Il a en outre été découvert de manière très surprenante que l'association de ces 3 composés augmente également la teneur en eau de la peau et/ou des muqueuses. Ainsi, l'association de ces 3 composés a l'avantage de procurer un effet hydratant complet, par l'augmentation de la teneur en eau d'une part et par le maintien de celle-ci en diminuant les pertes en eau d'autre part. Par ailleurs, les inventeurs ont découvert de manière très surprenante que cette association notamment lorsque les 3 composés sont ajoutés de façon concomitante, en particulier sous forme d'un pré-mélange dans une composition, permettait également d'augmenter et/ou de prolonger la teneur en ingrédients actifs cosmétiques et/ou pharmaceutiques, en particulier dermatologiques dans la peau et/ou les muqueuses, en particulier dans l'épiderme cutané.

La présente invention concerne donc l'association du pullulan ou d'un de ses dérivés estérifiés ou d'un de ses dérivés organominéraux à base de silicium avec un mélange de polysaccharides consistant en l'acide hyaluronique, un de ses sels ou dérivés estérifiés ou dérivés organominéraux à base de silicium et l'acide alginique, un de ses sels ou dérivés estérifiés ou dérivés organominéraux à base de silicium, le ratio en poids pullulan ou dérivés estérifiés ou dérivés organominéraux à base de silicium / acide hyaluronique, sels ou dérivés estérifiés ou dérivés organominéraux à base de silicium / acide alginique, sels ou dérivés estérifiés ou dérivés organominéraux à base de silicium étant compris dans la gamme de 1/1/1 à 1/10/10.

Au sens de la présente invention on entend par « association » le fait que les composés sont utilisés ensemble, préférentiellement sous la forme d'un pré-mélange. Avantageusement, il n'existe aucune liaison covalente entre les différents composés de l'association. Au sens de la présente invention, on entend par « pertes insensibles en eau », la diffusion passive et perspiration insensible mesurée au niveau cutanée ou mucosale. Egalement dénommée pertes transépidermiques en eau ou TEWL (transepidermal waterloss), les pertes insensibles en eau peuvent être mesurées par différentes méthodes notamment in vivo en particulier via un évaporimètre, un tewamètre, notamment in vitro, en particulier par la technique en cylindre ouvert décrite en exemple 1 et/ou ex vivo. Ainsi, par exemple, les pertes insensibles en eau sont mesurées par la technique décrite dans l'exemple 1 en cylindre ouvert et la diminution est déterminée par rapport à un témoin non traité. Cette technique s'inspire de celles décrites dans les publications Lieb et al. « A new in vitro method for transepidermal water loss : a possible method for moisturizer evaluation », J. Soc. Cosmet. Chem.,March 1988, 39, 107-109 et Strussmann et al. « Water waper permeability of skin care products in relation to molecular and environmental influence », Int. Jounal of Cosmetic Science, 1993, 15, 227-233. Néanmoins effectué sous étuve et donc en atmosphère sèche et à température élevée (45°C), ce test accentue et amplifie le phénomène de pertes insensibles en eau, permettant une mesure dans des conditions particulièrement sévères.
Au sens de la présente invention, on entend par « teneur en eau de la peau et/ou des muqueuses », la quantité d'eau contenue dans les épithélia, en particulier l'épiderme et/ou l'épithélium des muqueuses. Cette teneur traduit ainsi l'état d'hydratation. Il existe différentes méthodes de mesure de la teneur en eau de la peau et/ou des muqueuses, notamment in vivo et en particulier via le cornéomètre, et/ou par la mesure de la desquamation via le cornéofix, ou in vitro, notamment via la mesure de la conductivité diélectrique, en particulier selon la méthode décrite dans l'exemple 4.

Au sens de la présente invention, on entend par « teneur en ingrédients actifs cosmétiques et/ou pharmaceutiques dans la peau et/ou les muqueuses », la quantité d'ingrédients actifs cosmétiques et/ou pharmaceutiques, en particulier dermatologiques contenue dans les épithélia, en particulier l'épiderme et/ou l'épithélium des muqueuses. Il existe différentes méthodes de mesure de la teneur en ingrédients actifs dans la peau et/ou les muqueuses. Selon un mode préférentiel, cette teneur sera mesurée ex vivo, en particulier par étude de la cinétique de diffusion des ingrédients actifs dans la peau et/ou les muqueuses. Par exemple, il pourra s'agir de l'étude de pénétration par cellule de Franz, comme décrit à l'exemple 5.

Au sens de la présente invention, on entend par « ingrédient(s) cosmétique(s) et/ou pharmaceutique(s) » un ou des extraits végétaux et/ou une ou des molécules naturelles ou synthétiques et/ou leurs mélanges destiné à une application cosmétique et/ou pharmaceutique. Les ingrédients cosmétiques sont notamment définis par la nomenclature internationale des ingrédients cosmétiques (INCI).

On entend par « ingrédient actif cosmétique ou pharmaceutique », un ingrédient cosmétique ou pharmaceutique ayant une efficacité cosmétique et/ou pharmaceutique. Les ingrédients actifs pharmaceutiques correspondent aux principes actifs pharmaceutiques. Des catégories et exemples d'ingrédients actifs cosmétiques et /ou pharmaceutiques sont fournis ci-après.

De manière préférentielle, les ingrédients actifs cosmétiques et/ou pharmaceutiques selon la présente invention sont les composés du facteur d'hydratation naturelle (Natural Moisturizing Factor), tels que les acides aminés, l'urée, l'acide hyaluronique et le glycérol.

Au sens de la présente invention, on entend par « voie topique », l'application de l'association et/ou de la composition selon l'invention sur la surface de la peau et/ou des muqueuses, notamment par application directe ou par vaporisation.

Selon l'invention, on désigne par les muqueuses notamment la muqueuse buccale, labiale, nasale, oculaire, anale et/ou urogénitale, en particulier vaginale.

Au sens de la présente invention, le terme « véhicule cosmétique ou pharmaceutique approprié », signifie que la composition ou les composants de celle-ci sont adaptés à l'utilisation en contact avec la peau et/ou les muqueuses humaines sans toxicité, incompatibilité, instabilité, réponse allergique, ou leurs équivalents, indue.

Selon l'invention les « dérivés » sont donc les dérivés estérifiés et les dérivés organominéraux à base de silicium.

Au sens de la présente invention, on entend par « dérivés estérifiés de pullulan, d'acide hyaluronique ou d'acide alginique » tous les dérivés obtenus par estérification simple ou multiple d'une fonction alcoolique primaire ou secondaire ou d'une fonction acide du pullulan, de l'acide hyaluronique ou de l'acide alginique, et présentant sur la partie estérifiée une chaine carbonés comprenant de 1 à 6 atomes de carbones, avantageusement une chaine alkyle linéaire ou ramifiée.

Au sens de la présente invention, on entend par « dérivés organominéraux à base de silicium de pullulan, d'acide hyaluronique ou d'acide alginique » tous les dérivés qui contiennent au moins un silanol (-SiOH) et obtenus par condensation du pullulan, acide hyaluronique ou acide alginique avec une molécule de la famille des silanes.

Au sens de la présente invention, on entend par « sels d'acide hyaluronique, ou d'acide alginique », les composés ioniques qui résultent de la réaction de neutralisation de la forme acide de l'acide hyaluronique ou de l'acide alginique par un anion, notamment inorganiques, en particulier chlorure, sulfate, phosphate, sodique, potassique et magnésique.

Le pullulan est produit à partir de l'amidon par le champignon Aureobasidium pullulans et peut être obtenu à partir de différents ferments d'Aureobasidium pullulans. Selon la présente invention, le pullulan peut être utilisé sous sa forme aqueuse, éventuellement saline. Les dérivés de pullulan utilisables dans le cadre de la présente invention sont les dérivés cosmétiquement et/ou pharmaceutiquement acceptables, préférentiellement dermatologiquement acceptables, c'est-à-dire qu'ils sont non toxiques pour une administration sur l'être humain notamment par application topique et peuvent être appliqués sans risque et sans provoquer de réaction allergique ou inflammatoire en particulier sur la peau. Dans un mode de réalisation avantageux les dérivés de pullulan sont choisis parmi les dérivés organominéraux à base de silicium utilisés habituellement en cosmétique tels que par exemple ceux choisis parmi le triméthylsiloxysilylcarbamoyl pullulan et le triméthylsilyl pullulan. Avantageusement dans l'association, le pullulan ne se trouve pas sous la forme de dérivé. En particulier le pullulan a un poids moléculaire inférieur à 500 kDa, avantageusement d'environ 200 kDa. Le pullulan est disponible commercialement (Chemaster International, Chine ; Hayashibara, Japon).

Les sels et dérivés d'acide hyaluronique utilisables dans le cadre de la présente invention sont les sels ou dérivés cosmétiquement et/ou pharmaceutiquement acceptables, préférentiellement dermatologiquement acceptables. Avantageusement les sels d'acide hyaluronique sont choisis parmi l'hyaluronate de calcium hydrolysé, l'hyaluronate de sodium hydrolysé, l'hyaluronate de potassium, l'hyaluronate de sodium, l'hyaluronate sulfaté de sodium et leurs mélanges. Avantageusement il s'agit de l'hyaluronate de sodium. Dans un mode de réalisation avantageux, les dérivés de l'acide hyaluronique sont choisis parmi les dérivés utilisés habituellement en cosmétique tels que par exemple ceux choisis parmi les dérivés estérifiés, en particulier l'hyaluronate d'ascorbyle, l'hyaluronate de benzyle, l'hyaluronate de propylène glycol, l'hyaluronate acétylé de sodium, l'hyaluronate butyroyle de sodium ou l'hyaluronate d'hydroxypropyltrimonium, les dérivés organominéraux de silicium, en particulier l'hyaluronate de diméthylsilanol et leurs mélanges.
Avantageusement dans l'association l'acide hyaluronique se trouve sous la forme d'un de ses sels. De façon avantageuse il s'agit de l'hyaluronate de sodium. En particulier l'acide hyaluronique, ses sels ou dérivés estérifiés ont un poids moléculaire supérieur à 20 kDa, avantageusement compris entre 50 et 800 kDa, de façon avantageuse entre 250 et 450kDa. L'hyaluronate de sodium est disponible commercialement notamment auprès des sociétés Technidd Chemi-tech, Wuhan Fortuna Chemical, Dalian Chem Imp. and Exp. Group, Afine Chemicals Limited, Javenech SA, Soliance, Maprecos, Landy Enterprise Limited, Chandigarh Médical Corporation, Kartik Enterprises, DSA Exports.

Les sels et dérivés d'acide alginique utilisables dans le cadre de la présente invention sont les sels ou dérivés cosmétiquement et/ou pharmaceutiquement acceptables, préférentiellement dermatologiquement acceptables. Avantageusement les sels de l'acide alginique sont choisis parmi l'alginate d'ammonium, l'alginate de sodium, l'alginate de calcium, l'alginate de magnésium, l'alginate sulfate de sodium et l'alginate de potassium. Avantageusement il s'agit de l'alginate de sodium. Dans un mode de réalisation avantageux les dérivés de l'acide alginique sont choisis parmi les dérivés utilisés habituellement en cosmétique tels que par exemple ceux choisis parmi les dérivés estérifiés, notamment l'alginate de glycérile ou l'alginate de propylène glycol, les dérivés organominéraux de silicium de l'acide alginique, en particulier l'alginate de siloxanetriol ou l'alginate de méthylesilanol carboxyméthyl théophylline, et leurs mélanges. En particulier le dérivé est l'alginate de propylène glycol. Avantageusement dans l'association, l'acide alginique se trouve sous la forme d'un de ses sels. De façon avantageuse il s'agit de l'alginate de sodium. De façon avantageuse, l'acide alginique, ses sels ou dérivés, en particulier estérifiés ou organominéraux de silicium présentent un poids moléculaire compris entre 10 et 600 kDa, préférentiellement compris entre 30 et 550 kDa, encore préférentiellement de 100 à 550kDa. L'alginate de sodium est disponible commercialement notamment auprès des sociétés Laserson S.A, Univar, Danisco Ingrédients. BASF, BAM, Penta Manufacturing Company, Vevy Europe.

Avantageusement, l'acide alginique sera sous forme d'alginate de sodium, d'alginate de propylène glycol ou de leurs mélanges.

Dans un mode de réalisation, le ratio en poids pullulan, ou dérivés notamment estérifiés/ acide hyaluronique, sels ou dérivés notamment estérifiés de l'association selon l'invention est de 1/1.

Dans un mode de réalisation le ratio en poids pullulan ou dérivés notamment estérifiés/ acide alginique, sels ou dérivés notamment estérifiés de l'association selon l'invention est compris dans la gamme 1/1 - 1/2.

L'association contient donc un mélange de pullulan, éventuellement sous forme de dérivé, d'acide hyaluronique, éventuellement sous forme de sel ou dérivé notamment estérifié, de façon avantageuse sous forme d'hyaluronate de sodium, et d'acide alginique, éventuellement sous forme de sel ou dérivé notamment estérifié, de façon avantageuse sous forme d'alginate de sodium. Ce mode de réalisation présente l'avantage de diminuer très efficacement et rapidement les pertes insensibles en eau. Ainsi, il a notamment été démontré dans l'expérience selon l'exemple 3 menée in vitro en cylindre ouvert un effet de synergie sur la diminution des pertes insensibles en eau dès 4 heures après l'application de l'association et ce avec une intensité quasi identique à l'acide hyaluronique mais de manière beaucoup plus durable que ce dernier.

Selon ce mode de réalisation, de manière avantageuse, l'association comprend donc du pullulan, du hyaluronate de sodium et de l'alginate de sodium. Le ratio en poids de pullulan, éventuellement sous forme de dérivés / acide hyaluronique, sels ou dérivés estérifiés / acide alginique, sels ou dérivés estérifiés est compris dans la gamme 1/1/1 à 1/10/10, et encore préférentiellement dans la gamme 1/1/1 à 1/2/5. Selon un mode préférentiel, ce ratio est d'environ 1/1/2, encore préférentiellement il est de 1/1/2.

Outre les propriétés de limitation des pertes insensibles en eau, les inventeurs ont montré à l'exemple 3 que ce mode de réalisation permet d'augmenter la teneur en eau de la peau et/ou des muqueuses. Ainsi les inventeurs ont démontré que l'association des trois composés selon ce mode de réalisation permet à la fois d'augmenter la teneur en eau et de diminuer les pertes insensibles en eau, ce qui est particulièrement avantageux pour un effet hydratant complet, immédiat et durable au cours du temps. L'association selon ce mode de réalisation convient tout particulièrement comme agent hydratant pour la peau et/ou les muqueuses.

Par ailleurs, les inventeurs ont démontré que l'association selon ce mode de réalisation avait la propriété d'augmenter et/ou de prolonger la teneur en ingrédients actifs cosmétiques et/ou pharmaceutiques dans la peau et/ou les muqueuses et ce de manière durable (exemple 5). Cette propriété avantageuse de ladite association s'explique par la formation d'un réseau moléculaire pluristratifié, correspondant à un maillage moléculaire avec formation de liaisons hydrogène, lorsque les 3 composés pullulan, éventuellement sous forme de dérivés, acide hyaluronique, sels ou dérivés estérifiés, et acide alginique, sels ou dérivés estérifiés sont associés de façon concomitante, préférentiellement sous forme d'un pré-mélange, à un ratio se trouvant dans la gamme 1/1/1 à 1/10/10, et encore préférentiellement dans la gamme 1/1/1 à 1/2/5, avantageusement un ratio de 1/1/2 (exemple 6).

La présente invention concerne en outre l'utilisation de l'association selon la présente invention en tant qu'agent hydratant de la peau et/ou des muqueuses, avantageusement ayant une action immédiate, et durable. En effet les inventeurs se sont aperçus que l'association comprenant trois composants avait une activité renforcée sur la diminution des pertes en eau et donc sur le maintien de l'hydratation, en raison de la synergie entre ses différents composants, et ce pour une longue durée. En effet, l'effet synergique semble d'autant plus important en fonction du temps. Avantageusement cette activité dure au moins 5 heures, préférentiellement 10 heures, encore préférentiellement 24 heures et peut aller même jusqu'à 48 heures. En outre, les inventeurs se sont aperçus que cette association comprenant les trois composants avait une activité renforcée pour l'augmentation de la teneur en eau, de manière durable, et ce notamment en raison de la formation d'un réseau moléculaire lorsque les 3 composés pullulan, éventuellement sous forme de dérivés, acide hyaluronique, sels ou dérivés estérifiés, et acide alginique, sels ou dérivés estérifiés sont associés de façon concomitante, préférentiellement sous forme d'un pré-mélange, à un ratio se trouvant dans la gamme 1/1/1 à 1/10/10, et encore préférentiellement dans la gamme 1/1/1 à 1/2/5, avantageusement un ratio de 1/1/2 (exemple 6). Avantageusement cette activité dure au moins 3 heures, encore préférentiellement 10 heures. La présente invention concerne donc l'utilisation de l'association selon la présente invention pour diminuer les pertes insensibles en eau et/ou pour augmenter la teneur en eau de la peau et/ou des muqueuses.

La présente invention concerne de plus l'utilisation de l'association selon la présente invention pour augmenter et/ou prolonger la teneur en ingrédients actifs cosmétiques et/ou pharmaceutiques dans la peau et/ou les muqueuses et/ou la maintenir dans le temps.

L'association selon l'invention est préférentiellement utilisée seule ou sous la forme d'un mélange d' ingrédients cosmétiques ou pharmaceutiques contenu dans un véhicule cosmétique ou pharmaceutique, notamment dermatologique approprié à sa formulation et/ou à son intégration dans une composition cosmétique ou pharmaceutique, notamment dermatologique. L'association peut également être utilisée dans une composition cosmétique ou pharmaceutique, notamment dermatologique, c'est-à-dire en combinaison avec un véhicule cosmétique ou pharmaceutique approprié, notamment dermatologique approprié, et préférentiellement en application topique. Ladite association consiste ainsi en un mélange des 3 composés pullulan, éventuellement sous forme de dérivés, acide hyaluronique, sels ou dérivés estérifiés, et acide alginique, sels ou dérivés estérifiés à un ratio se trouvant dans la gamme 1/1/1 à 1/10/10, et encore préférentiellement dans la gamme 1/1/1 à 1/2/5, avantageusement un ratio de 1/1/2 (exemple 6)..

L'association selon l'invention peut également être utilisée sous la forme d'une composition d'entretien, en particulier composition détergente, comme par exemple un nettoyant multi-usages, un savon et/ou un liquide vaisselle.

Selon un premier mode de réalisation, la présente invention concerne un mélange d'ingrédients cosmétiques ou pharmaceutiques, en particulier dermatologiques, avantageusement destinés à être incorporés dans une composition cosmétique ou pharmaceutique, en particulier dermatologique, comprenant l'association selon la présente invention en une teneur comprise entre 0,1 et 5% en poids de matière sèche par rapport au poids total du mélange d'ingrédients, avantageusement entre 1 et 3% en poids et un véhicule cosmétique ou pharmaceutique approprié.

Avantageusement, le véhicule du mélange d'ingrédients cosmétiques ou pharmaceutiques est et/ou contient de l'eau.

Ainsi l'association est comprise dans le mélange d'ingrédients cosmétiques ou pharmaceutiques, en particulier dermatologiques, à une teneur de 0,1 et 5% en poids de matière sèche par rapport au poids total du mélange d'ingrédients cosmétiques ou pharmaceutiques, en particulier entre 0,25 et 3% en poids, plus particulièrement entre 1 et 3%, encore plus particulièrement 1 % en poids. Selon un mode avantageux, le mélange d'ingrédients cosmétiques ou pharmaceutiques, notamment dermatologique contient :
- le pullulan, un de ses dérivés cosmétiquement ou pharmaceutiquement acceptable provenant de l'association selon la présente invention en une teneur comprise entre 0,01 et 3% en poids de matière sèche par rapport au poids total du mélange d'ingrédients cosmétiques ou pharmaceutiques, en particulier entre 0,1 et 1% en poids, encore plus particulièrement entre 0,25 et 0,5% en poids, très particulièrement 0,25% en poids et
- l'acide hyaluronique, un de ses sels ou dérivés cosmétiquement ou pharmaceutiquement acceptable provenant de l'association selon la présente invention en une teneur comprise entre entre 0,01 et 3% en poids de matière sèche par rapport au poids total du mélange d'ingrédients cosmétiques ou pharmaceutiques, en particulier entre 0,1 et 1 % en poids, plus particulièrement entre 0, 1 et 1 % en poids, encore plus particulièrement entre 0,25 et 0,5% en poids, très particulièrement 0,25% en poids et
- l'acide alginique, un de ses sels ou dérivés cosmétiquement ou pharmaceutiquement acceptable provenant de l'association selon la présente invention en une teneur comprise entre 0,01 et 3% en poids de matière sèche par rapport au poids total du mélange d'ingrédients cosmétiques ou pharmaceutiques, de façon encore plus avantageuse entre 0,1 et 2 % en poids, encore plus particulièrement 0,5% en poids.

Le mélange d'ingrédients cosmétiques ou pharmaceutiques contient l'association du pullulan ou d'un de ses dérivés, de l'acide hyaluronique, ou d'un de ses sels ou dérivés et de l'acide alginique ou d'un de ses sels ou dérivés à un ratio compris entre 1/1/1 et 1/10/10, préférentiellement compris entre 1/1/1 et 1/2/5, encore préférentiellement à un ratio de 1/1/2.

Le mélange d'ingrédients cosmétiques ou pharmaceutiques, en particulier dermatologiques, peut comprendre en outre un autre agent hydratant, ayant avantageusement un effet synergique ou complémentaire. Il est en particulier choisi parmi le tréhalose, la sérine, l'urée et leurs mélanges.
Selon un mode particulièrement avantageux, le mélange d'ingrédients cosmétiques ou pharmaceutiques, en particulier dermatologiques comprend au moins l'association selon l'invention ainsi que la sérine, le tréhalose, l'urée et de l'eau.
Le mélange d'ingrédients cosmétiques ou pharmaceutiques, en particulier dermatologiques, peut être utilisé dans une composition cosmétique ou pharmaceutique, en particulier dermatologique, préférentiellement à une teneur en poids de matière sèche par rapport au poids total de la composition comprise entre 0,1 et 10%, avantageusement entre 0,1 et 5%, en particulier entre 1 à 3%.

Dans un deuxième mode de réalisation de la présente invention, la présente invention concerne une composition cosmétique ou pharmaceutique, en particulier dermatologique, destinée à une administration par voie topique comprenant l'association en une teneur comprise entre 0,0001% et 20% en poids de matière sèche par rapport au poids total de la composition, avantageusement entre 0,01 et 5% en poids, ou le mélange d'ingrédients cosmétiques ou pharmaceutiques selon la présente invention et un véhicule cosmétique ou pharmaceutique approprié.

Ainsi dans ce second mode de réalisation de la présente invention, l'association est comprise dans une composition cosmétique ou pharmaceutique, en particulier dermatologique, en particulier destinée à être administrée à un être humain, préférentiellement par application topique, préférentiellement cutanée. L'association selon la présente invention est alors comprise dans la composition à une teneur comprise entre 0,0001% et 20% en poids de matière sèche total par rapport au poids total de la composition, plus avantageusement entre 0,005 et 10% en poids, encore plus avantageusement entre 0,01 et 5% en poids, en particulier entre 0,02 et 1% en poids, plus particulièrement 0,03 % en poids. La composition cosmétique ou pharmaceutique, en particulier dermatologique contient également un véhicule et/ou excipient cosmétique et/ou pharmaceutique, notamment dermatologique.

Selon un mode avantageux, la composition cosmétique ou pharmaceutique, notamment dermatologique contient :
- le pullulan, un de ses dérivés cosmétiquement ou pharmaceutiquement acceptable en une quantité comprise entre 0,0001 et 10% en poids de matière sèche par rapport au poids total de la composition, et de façon avantageuse une quantité entre 0,001% et 5 % en poids, de façon plus avantageuse entre 0,001 et 3% en poids, en particulier entre 0,005 et 0,1% en poids, encore préférentiellement entre 0,007 et 0,02% en poids, et
- l'acide hyaluronique, un de ses sels ou dérivés cosmétiquement ou pharmaceutiquement acceptable en une teneur comprise entre 0,0001 et 10% en poids de matière sèche par rapport au poids total en matière sèche de la composition, avantageusement entre 0,001% et 5 % en poids, plus avantageusement entre 0,001 et 3% en poids, en particulier entre 0,005 et 0,1% en poids, encore préférentiellement entre 0,007 et 0,02% en poids et
- l'acide alginique, un de ses sels ou dérivés cosmétiquement ou pharmaceutiquement acceptable en une teneur comprise entre 0,0001 et 10% en poids de matière sèche par rapport au poids total de la composition, avantageusement entre 0,001% et 5 % en poids, plus avantageusement entre 0,001 et 1% en poids, en particulier entre 0,007 et 0,2% en poids, encore plus particulièrement entre 0,01 et 0,1% en poids.

Selon un mode préférentiel, la composition cosmétique ou pharmaceutique contient l'association du pullulan ou d'un de ses dérivés, de l'acide hyaluronique, ou d'un de ses sels ou dérivés et de l'acide alginique ou un de ses sels ou dérivés, préférentiellement à un ratio de 1/1/2.

La composition selon la présente invention, sous la forme de composition cosmétique ou pharmaceutique ou d'un mélange d'ingrédients cosmétiques ou pharmaceutiques, peut se présenter sous toutes les formes galéniques classiquement utilisées pour une application topique, telles que les formes liquides ou solides ou même sous la forme de liquide sous pression. Elles peuvent notamment être formulées sous la forme d'une solution, aqueuse ou huileuse, une crème ou un gel aqueux ou un gel huileux, notamment en pot ou en tube, notamment un gel douche, un shampoing, un lait, une émulsion, un hydrogel, une microémulsion ou une nanoémulsion, notamment huile-dans-eau ou eau-dans-huile ou multiple ou siliconée, un sérum, une lotion, notamment en flacon de verre, de plastique ou en flacon doseur ou en aérosol, une ampoule, un savon liquide, une pâte, un pain dermatologique, une pommade, une mousse, un masque, un patch, un produit anhydre, de préférence liquide, pâteux ou solide, par exemple sous forme de bâtonnet notamment en stick ou en poudres, notamment de maquillage. En particulier la composition se présente sous la forme d'un sérum, d'une lotion, d'une crème, d'un lait, d'une pommade, d'une pâte, d'une mousse, d'une émulsion, d'un hydrogel, d'un gel douche, d'un masque, d'un stick d'un patch, ou de poudres de maquillage, avantageusement d'une crème ou d'une lotion.

Les compositions selon l'invention peuvent contenir tout solvant approprié et/ou tout véhicule approprié et/ou tout excipient approprié, éventuellement en combinaison avec d'autres composés d'intérêts. Elles peuvent notamment contenir un excipient cosmétiquement ou dermatologiquement acceptable choisis parmi des agents tensioactifs, des conservateurs, des agents tampon, des agents gonflants, des agents chélatants, des agents biocides, des dénaturants, des agents opacifiants, des ajusteurs de pH, des agents réducteurs, des agents stabilisants, des émulsifiants, des épaississants, des gélifiants, des polymères filmogènes, des solvants, des charges, des bactéricides, des absorbeurs d'odeurs, des agents matifiants, des agents conditionneurs, des agents de texture, des agents de brillance, des pigments, des colorants, des parfums et des filtres solaires chimiques ou minéraux, des oligo-éléments, des huiles essentielles. Ces combinaisons sont également couvertes par la présente invention. Le CTFA Cosmetic Ingrédient Handbook, Second Edition (1992) décrit différents ingrédients cosmétiques et pharmaceutiques utilisés couramment dans l'industrie cosmétique et pharmaceutique, qui sont en particulier adaptés à une utilisation topique.

Différents ingrédients actifs cosmétiques et /ou pharmaceutiques, en particulier dermatologiques, seuls ou en mélange, peuvent être délivrés par voie topique en utilisant l'association selon la présente invention.

Les compositions selon l'invention peuvent ainsi contenir des ingrédients actifs cosmétiques ou pharmaceutiques, notamment dermatologiques conduisant à un effet complémentaire ou éventuellement synergique tels que des actifs hydratants, des actifs anti-âge, des actifs anti radicalaires et/ou des eaux thermales. Il peut ainsi s'agir par exemple d'agents dépigmentants, éclaircissants ou anti-pigmentants de la peau, d'agents de coloration de la peau ou pro-pigmentants, d'inhibiteurs de NO-synthase, d'agents anti-séborrhéiques pour le soin des peaux grasses, d'agents stimulant la synthèse de macromolécules dermiques ou épidermiques, notamment de la matrice extracellulaire et/ou empêchant leur dégradation, d'agents stimulant la prolifération des fibroblastes ou des kératinocytes et/ou la différenciation des kératinocytes, d'agents myorelaxants ou dermo-décontractants, d'agents antimicrobiens, d'agent tenseurs, d'agents antipollutions ou anti-radicalaires, d'agents apaisants, calmants ou relaxants, d'agents agissant sur la microcirculation pour améliorer l'éclat du teint, en particulier du visage, d'agents photoprotecteurs, d'agent desquamants, d'agents cicatrisants, d'agents amincissants, d'agents anti âge ou éventuellement d'autres agents hydratants et/ou renforçant la barrière épidermique pour renforcer encore l'activité hydratante.

Les actifs hydratants, émollients ou humectants conduisant à un effet complémentaire ou éventuellement synergique sont ceux qui renforcent la fonction barrière et diminuent les pertes insensibles en eau et/ou ceux qui augmentent la teneur en eau de la peau et/ou des muqueuses ou stimulent l'activité sécrétoire des glandes sébacées et/ou stimulent la synthèse d'aquaporine pour améliorer la circulation de l'eau dans les cellules. On peut citer à titre d'exemple non limitatif les actifs suivants : la sérine, l'urée et ses dérivés, les produits commercialisés sous le nom de Marine Filling sphères™, Advances moisturizing complex™, Hyaluronic Filling Spheres™, vegetal filling spheres™ Osmogelline™, Micropatch™, les alkylcelluloses, les lécithines, les composés à base de sphingoïde, les céramides, les phospholipides, le cholestérol et ses dérivés, les glycosphingolipides, les phytostérols (stigmastérol et béta-sitostérol, campestérol), les acides gras essentiels, le 1-2 diacylglycérol, la 4 - chromanone, les triterpènes pentacycliques tels que l'acide ursolique, la vaseline, la lanoline, les sucres en particulier le tréhalose et ses dérivés, le rhamnose, fructose, maltose, lactose, erythritol, le mannitol, le D-xylose et le glucose, l'adénosine et ses dérivés, le sorbitol, les alcools polyhydriques, avantageusement en C2-C6, et de façon encore avantageuse en C3-C6, tels que la glycérine, le propylène glycol, le 1,3-butylène glycol, le dipropylène glycol, la diglycérine, la polyglycérine et leur mélange, le glycérol et ses dérivés, le polyacrylate de glycérol, le lactate de sodium, le pentanediol, la serine, les acides lactiques, les AHA, les BHA, le pidolate de sodium, le xylitol, le lactate de sodium, l'ectoine et ses dérivés, le chitosane et ses dérivés, le collagène, le plancton, les dérivés stéroïdiens (dont la DHEA, ses dérivés 7-oxydés et/ou 17-alkylés et les sapogénines), le dihydrojasmonate de méthyle, la vitamine D et ses dérivés, un extrait de Malva sylvestres ou un extrait de Centella asiatica, des homopolymères d'acide acrylique, le beta-glucan et en particulier le sodium carboxymethyl beta-glucane,, un dérivé C-glycoside tel que ceux décrits dans la demande WO 02/051828, une huile de rosier muscat, un extrait de micro-algue Prophyridium cruentum enrichi en zinc commercialisé par Vincience sous la dénomination Algualane Zinc™, l'arginine, l'acetyl hexapeptide commercialisé par Lipotech sous le nom Diffuporine™, l'hydralysat de Viola tricolor commercialisé par Silab sous le nom Aquaphyline™.
Les autres agents actifs conduisant à un effet complémentaire peuvent être de façon non limitative choisis dans la liste d'agents dépigmentants, éclaircissants ou anti-pigmentants de la peau, d'agents de coloration de la peau ou pro-pigmentants, d'inhibiteurs de NO-synthase, d'agents anti-séborrhéiques pour le soin des peaux grasses, d'agents stimulant la synthèse de molécules et macromolécules dermiques ou épidermiques et/ou empêchant leur dégradation, d'agents stimulant la prolifération des fibroblastes ou des kératinocytes et/ou la différenciation des kératinocytes, d'agents myorelaxants ou dermo-décontractants, d'agents antimicrobiens, d'agent tenseurs, d'agents antipollutions ou anti-radicalaires, d'agents apaisants, calmants ou relaxants, d'agents agissant sur la microcirculation pour améliorer l'éclat du teint, en particulier du visage, d'agents stimulant la synthèse de la matrice extracellulaire, d'agents photoprotecteurs, d'agents desquamants, d'agents cicatrisants et/ou d'agents anti âge.

Des exemples de ces agents utilisables dans les compositions selon la présente invention et/ou qui peuvent être délivrés par l'association selon la présente invention sont les suivants :
Comme exemples d'agents cicatrisants, on peut citer notamment : l'allantoïne, l'urée, certains acides aminés comme l'hydroxyproline, l'arginine, la sérine, les extraits de levures, le chitosane et dérivés comme le glutamate de chitosane, des extraits de millefeuilles, l'acide folique, des bétaglucan et dérivés, du beurre de karité et ses fractions purifiées, les exopolysaccharides modifiés et les polyaminosaccharides alkylsulfonnés.

L'actif peut également être choisi parmi les agents anti-âges, c'est-à-dire ayant notamment un effet restructurant de la barrière cutanée, les agents prévenant et/ou diminuant la glycation des protéines de la peau, en particulier le resvératrol, en particulier des protéines du derme, telles que le collagène, les actifs stimulant le métabolisme énergétique des cellules et leurs mélanges, un agent à action globale anti-âge, notamment anti-taches pigmentaires en particulier la niacinamide ou vitamine B3 et dérivés. L'agent ayant un effet restructurant de la barrière cutanée peut être choisi parmi un des extraits de levure comme le Relipidium™ de BASF Beauty Care Solutions France SAS, des sphingosines comme la salicyloyl sphingosine, un mélange de xylitol, de xylityl polyglycoside et de xylitan, des extraits de solanacée comme le Lipidessence™ de BASF Beauty Care Solutions France SAS et leurs mélanges. On peut encore citer notamment les céramides, les composés à base de sphingoïdes, les glycosphingolipides, les phospholipides, le cholestérol et ses dérivés, les phytostérols, les acides gras essentiels, le diacylglycérol, la 4-chromanone et dérivés de chromone et leurs mélanges, vitamine B5 ou pantothenate et dérivés

L'actif stimulant le métabolisme énergétique des cellules peut par exemple être choisi parmi la biotine, un mélange de sels de sodium, de manganèse, de zinc et de magnésium d'acide pyrrolidone carboxylique, un mélange de gluconate de zinc, de cuivre et de magnésium et leurs mélanges.

Comme exemple d'agents dépigmentants, éclaircissants ou anti-pigmentants de la peau utilisables dans le cadre de la présente invention on peut citer notamment les composés suivants : l'acide kojique, l'acide ellagique, l'acide férulique et ses dérivés, l'arbutine l'hydroquinone, le pantothenosulfonate de calcium, la boldine, la diacetylboldine, l'acide ascorbique et ses dérivés, notamment le glucoside d'ascorbyle.

Les agents de coloration de la peau ou pro-pigmentants peuvent être notamment choisis parmi les agents favorisant la mélanogénèse, les agents de coloration artificielle de la peau, et leurs mélanges.
Les agents favorisant la mélanogénèse sont notamment choisis parmi :
- les substrats de la biosynthèse de la mélanine, notamment la L-tyrosine et ses dérivés, la L- dihydrophénylalanine ;
- les agents capables de stimuler la synthèse de mélanine, et en particulier les complexes d'ions métalliques tels que le cuivre et de peptones tels qu'un hydrolysat protéique provenant du soja, du collagène ou de caséine, comme décrits dans le brevet US5698184 ;
- les agents capables de stimuler l'activité ou l'expression de la tyrosinase, éventuellement par augmentation du taux d'AMPc intra-cellulaire tels que, notamment les peptides pro-opiomélanocortiques ; les alpha-MSH ou analogues d'alpha-MSH; ou les agonistes des récepteurs MC1R (US5683981, WO9825584), les analogues de l'AMPc, la Forskoline et les bases xanthiques (par exemple la caféine ou la théophylline) ou par activation de la protéine kinase C tels que les diacylglycérols ou bien les psoralènes,
- les agents capables de stimuler le transfert des mélanosomes des mélanocytes vers les kératinocytes, par exemple en stimulant les récepteurs PAR-2 tels que citer les polyphénols catéchiques, en particulier la catéchine, épicatéchine, gallocatéchine, épigallocatéchine, leurs sels et leurs esters, sous forme monomères ou oligomères, ainsi que les extraits végétaux en contenant, en particulier les extraits de thé vert.
Les agents de coloration artificielle de la peau sont notamment choisis parmi :
- les agents autobronzants, tels que l'isatine, l'alloxane, la ninhydrine, le glycéraldéhyde, l'aldéhyde mésotartrique, la glutaraldéhyde, l'érythrulose, la dihydroxyacétone (DHA),
- les agents de coloration additionnels tels que les extraits de sorgho et un extrait de Chicorium intybus, en particulier en combinaison avec un extrait de Gymnema sylvestre ou muirapuama, commercialisés sous le nom de QUICKSUN par BASF Beauty Care Solutions France SAS.
Des exemples d'inhibiteurs de NO-synthase sont notamment un extrait de végétal de l'espèce Vitis vinifera.

L'agent anti-séborrhéique dans la composition selon l'invention peut être un inhibiteur de 5α-réductase, tel que les rétinoïdes, la sarcosine, les sels de zinc, en particulier le gluconate de zinc, le salicylate de zinc, l'acide azélaique et/ou leurs dérivés, et/ou leurs mélanges et un extrait d'Orthosiphon stamineus commercialisé sous le nom de MAT XS™ bright par BASF Beauty Care Solutions France SAS. La composition peut également contenir un agent absorbeur de sébum, en particulier un talc et/ou un polymère absorbant, un agent antibactérien notamment ceux décrit dans la demande de brevet FR2863893, et en particulier un extrait de Boldo, un tel extrait étant notamment commercialisé par la Demanderesse sous le nom Betapur™, un agent comédolytique en particulier l'acide rétinoique et un de ses dérivés tels que isotrétinoine, adapalène et/ou acide 13cis retinoique et le péroxyde de benzoyle, un agent antibiotique local, en particulier l'érythromycine et/ou le phosphate de clindamycine et leurs mélanges.
Parmi les actifs stimulant la synthèse des macromolécules du derme ou empêchant leur dégradation, on peut citer ceux qui agissent comme :
- un agent stimulant la synthèse de fibronectine, en particulier un extrait de maïs, un tel extrait étant notamment commercialisé par la Demanderesse sous le nom Deliner™ et le palmitoyl pentapeptide commercialisé par la société SEDERMA sous la dénomination commerciale Matrixil®,
- un agent de protection du facteur de croissance des fibroblastes (FGF2) de la matrice extracellulaire contre sa dégradation et/ou sa dénaturation, notamment un extrait d'*Hibiscus Abelmoscus* tel que décrit dans la demande de brevet au nom de la Demanderesse déposée sous le numéro FR0654316 et/ou un agent de stimulation de croissance des fibroblastes par exemple un extrait de soja fermenté contenant des peptides, connu sous le nom de Phytokine™ commercialisé par la Demanderesse et également décrit dans la demande de brevet EP1119344 B1 (Laboratoires Expanscience), et préférentiellement une combinaison de ces deux extraits ;
- un agent stimulant la synthèse de laminine, en particulier un extrait de malt modifié par biotechnologie, un tel extrait étant notamment commercialisé par la Demanderesse sous le nom Basaline™ ;
- un agent stimulant l'expression et/ou l'activité de la Hyaluronane synthase 2 (HAS2) tels que les extraits végétaux décrits dans la demande de brevet FR2 893 252 A1 et en particulier un extrait aqueux de Galanga (*Alpinia galanga*) *;*
- un agent stimulant la synthèse de lysyl oxydase like (LOXL) tel que un extrait de Geophila cordifolia et ceux décrits dans la demande de brevet FR2855968, et en particulier un extrait d'aneth ;
- un agent stimulant la synthèse d'ATP intracellulaire, notamment un extrait d'algue *Laminaria digitata ;*
- un actif stimulant la synthèse des glycosaminoglycanes, tels que le produit de fermentation du lait ;
- un actif stimulateur de collagène tel que le rétinol et/ou la vitamine C ;
- un actif inhibiteur des métalloprotéinases (MMP) telles que plus particulièrement les MMP 1, 2, 3, 9 tel que les rétinoïdes et dérivés, les oligopeptides et les lipopeptides, les lipoaminoacides, l'extrait de malt commercialisé par BASF Beauty Care Solutions France sous la dénomination commerciale Collalift®, l'extrait hydrolysé de pomme de terre commercialisé sous le nom Extracellium™ par BASF Beauty Care solutions France SAS; le lycopène ; les isoflavones, la quercetine, le kaempferol, l'apigenine .
Les agents stimulant la prolifération des kératinocytes, utilisables dans la composition selon l'invention, comprennent notamment les rétinoïdes tels que le rétinol et ses esters, dont le palmitate de rétinyle et le phloroglucinol. Les agents stimulant la différenciation des kératinocytes comprennent par exemple les minéraux tels que le calcium et les lignanes tels que le sécoisolaricirésinol ainsi que l'extrait d'Achillea millefollium commercialisé sous le nom de Neurobiox™ par BASF Beauty Care Solutions France.
Les agents myorelaxants ou dermo-décontractants utilisables dans la composition selon l'invention comprennent le gluconate de manganèse, le Diazepam, certaines amines secondaires et tertiaires carbonylées, l'adénosine, ainsi que les sapogénines.

Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octoxyglycérine, l'octanoylglycine, le caprylyl glycol, l'acide 10-hydroxy-2-décanoïque, , le farnesol, les phytosphingosines et leurs mélanges.
Parmi les agents tenseurs utilisables dans la composition selon la présente invention, on peut citer notamment les polymères synthétiques, tels que les latex de polyuréthanne ou les latex acryliques ; les polymères d'origine naturelle, notamment les polyholosidessous forme d'amidon ou sous forme de carraghénanes, alginates, agars, gellanes, polymères cellulosiques et pectines; les protéines et hydrolysats de protéines végétales de soja ; les silicates mixtes ; les microparticules de cire ; les particules colloïdales de charge inorganique choisies par exemple parmi la silice, les composites silice-alumine ; ainsi que leurs mélanges.
La composition peut comprendre des agents dits antipollution, en particulier piégeur d'ozone que sont par exemple la vitamine C et ses dérivés dont le glucoside d'ascorbyle; les phénols et polyphénols, en particulier les tannins, l'acide ellagique et l'acide tannique; l'épigallocatéchine et les extraits naturels en contenant, en particulier les extraits de thé vert; les anthocyanes; les acides phénols, les stilbènes, le resvératrol; des actifs piégeurs de composés aromatiques mono- ou polycycliques les tannins tels que l'acide ellagique et les dérivés indoleset/ou des actifs piégeurs de métaux lourds tels que l'EDTA, des actifs anti-radicalaires tels que la vitamine E et ses dérivés tels que l'acétate de tocophéryle ; les bioflavonoïdes; la coenzyme Q10 ou ubiquinone.

Comme agents apaisants utilisables dans la composition selon l'invention, on peut citer : les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de Pueraria lobata commercialisé sous le nom Inhipase® par BASF Beauty Care Solutions France SAS, les extraits de Theobroma cacao.
Les ingrédients actifs agissant sur la microcirculation (vasoprotecteurs ou vasodilatateurs) peuvent être choisis parmi les flavonoïdes, les ruscogénines, les nicotinates, les huiles essentielles.

Les ingrédients actifs photoprotecteurs ou filtres UVA et/ou UVB utilisables selon la présente invention sont notamment les agents photoprotecteurs actifs dans l'UV-A et/ou l'UV-B, tels que les dérivés de l'acide para-aminobenzoïique notamment « UVINUL P25 » par BASF, les dérivés salicyliques en particulier l'homosalate seul ou en association avec des oxydes de titane
les dérivés du dibenzoylméthane, les dérivés cinnamiques, les dérivés de diphénylacrylate, dont Octocrylene vendu notamment sous le nom commercial « UVINUL N539 » par BASF, les dérivés de la benzophénone, notamment Benzophenone-1 vendue notamment sous le nom commercial « UVINUL 400 » par BASF, les dérivés du benzylidène camphre, les dérivés de benzimidazole, les dérivés de triazine, dont Ethylhexyl triazone vendu notamment sous le nom commercial «UVINUL T150 » par BASF, , les dérivés de benzotriazole, les dérivés anthranilique, les dérivés d'imidazolines les dérivés de benzalmalonate, les dérivés de 4,4-diarylbutadiène, et leurs mélanges.
Par "agent desquamant", on entend tout composé capable d'agir :
- soit directement sur la desquamation en favorisant l'exfoliation, tels que les β-hydroxyacides, en particulier l'acide salicylique et ses dérivés en particulier ester (dont l'acide n-octanoyl 5-salicylique), les acides de fruits, les α-hydroxyacides, tels que l'acide salicylique, les acides glycoliques, citriques, lactiques, tartriques, maliques ou mandéliques, l'acide gentisique ou ses esters en particulier le gentisate de tocopherol, les oligofucoses, l'acide cinnamique, le resvératrol et certains dérivés d'acide jasmonique, et/ou leurs dérivés et/ou leurs mélanges,
- soit sur les enzymes impliquées dans la desquamation ou la dégradation des cornéodesmosomes, les glycosidases, les agents chélatants des sels minéraux : l'EDTA,les composés aminosulfoniques, le méthyl glycine diacétate de sodium commercialisé par BASF sous la dénomination commerciale TRILON™, le miel, les dérivés de sucre tels que l'O-octanoyl-6-D-maltose et la N-acétyl glucosamine.

Les actifs procurant un effet de bien-être tels que ceux mimant les effets des béta-endorphines pour améliorer la fonction barrière de la peau, tels que ceux cités dans la demande de brevet US 2006069032 ; les actifs stimulants la synthèse des béta-endorphine tels que un extrait de la plante Tephrosia purpurea.

Les actifs amincissants peuvent être notamment choisis parmi : les agents inhibiteurs de la lipoprotéine lipase tels que ceux décrits dans le brevet US2003086949 (Coletica) et en particulier un extrait de liane du Pérou (Uncaria tomentosa); les actifs drainants, notamment l'hesperitine laurate (Flavagrum®), or quercitine caprylate(Flavenger®); les agents inhibiteurs de l'enzyme phosphodiestarase, les agents activateurs de l'adenylate cyclase, l'AMPc et/ou les actifs capable de piéger la spermine et/ou la spermidine. On peut citer à titre d'exemple de ces actifs un extrait de racine de Coleus Forskohlii, un extrait de cecropia obtusa, de Uva lactuca, la caféine, la forskoline, la théophylline, la théobromine et/ou leurs dérivés, un produit de kappa carraghénanes hydrolysé dénommé Slimexcess™ commercialisé par BASF Beauty Care Solutions France SAS et/ou leurs mélanges.

Tous ces ingrédients actifs cosmétiques ou pharmaceutiques peuvent être utilisés avec l'association selon la présente invention, en particulier pour augmenter leur teneur dans la peau et/ou les muqueuses et en prolonger les effets, notamment via un effet retard sur leurs diffusions percutanées par l'intermédiaire du réseau moléculaire formé lorsque les 3 composés selon l'invention sont associés de façon concomitante, et préférentiellement sous forme de pré-mélange, dans les compositions selon l'invention.

De manière préférentielle, les compositions selon l'invention sous forme d'un mélange d'ingrédients cosmétiques ou pharmaceutiques ou de composition cosmétique ou pharmaceutique contiennent au moins ingrédients actifs cosmétiques ou pharmaceutiques, en particulier dermatologiques, et préférentiellement de un à cinq ingrédients cosmétiques, encore préférentiellement de un à trois ingrédients actifs cosmétiques ou pharmaceutiques, en particulier dermatologiques. Préférentiellement, l'ingrédient actif est choisi parmi ceux précédemment cités, avantageusement parmi les agents anti-âge et hydratants, et encore préférentiellement parmi l'urée, le tréhalose, la sérine, la taurine et/ou un de ses dérivés, l'inositol, la betaine, en particulier au moins deux de ces composés, préférentiellement choisis parmi la sérine, l'urée et le tréhalose et avantageusement trois de ces composés, préférentiellement la sérine, l'urée et le tréhalose.

La présente invention concerne en outre l'utilisation de la composition cosmétique selon la présente invention pour diminuer les pertes insensibles en eau, pour augmenter la teneur en eau de la peau et/ou muqueuses, pour maintenir ou renforcer l'état d'hydratation de la peau et/ou muqueuse, et/ou pour prévenir ou ralentir l'apparition des signes de sécheresse cutanée et/ou mucosale, en particulier pendant au moins 10 heures, avantageusement 24 heures, en particulier jusqu'à 48 heures et/ou pour le traitement des états de sècheresse cutanée et/ou mucosale tels que et/ou les tiraillements associées aux peaux ou muqueuses sèches et/ou pour prévenir ou diminuer l'apparition des rides liées à une sécheresse cutanée, et/ou pour améliorer le confort des peaux et/ou muqueuses sèches, et/ou pour le traitement des peaux et/ou muqueuses d'aspect rugueux à la vue et/ou au toucher.

L'association selon l'invention, notamment sous la forme d'une composition cosmétique ou pharmaceutique, en particulier dermatologique convient donc tout particulier pour le soin et/ou le traitement des peaux et/ou muqueuses sèches, des peaux et/ou muqueuses agressées, sensibles, sensibilisées, altérées, intolérantes, séniles, fragiles ou réactives. L'association convient également tout particulièrement pour une utilisation sur les muqueuses buccales, labiales, oculaires, génitales, notamment vaginales, seule ou sous forme de composition cosmétique ou dermatologique, en particulier pour en améliorer l'hydratation. L'association seule ou sous forme de composition ou de mélange d'ingrédients cosmétique ou pharmaceutique convient également tout particulièrement pour la réalisation de compositions de soin ophtalmique.

L'association seule ou sous la forme de composition cosmétique ou pharmaceutique ou de mélange d'ingrédients cosmétiques ou pharmaceutiques peut être appliquée notamment sur le visage, le cou, le buste, le corps, les mains, les pieds, le cuir chevelu, les yeux et/ou les lèvres.

La présente invention concerne en outre une composition pharmaceutique, en particulier dermatologique, selon la présente invention pour utilisation dans le traitement et/ou la prévention des gerçures et/ou des dartres ou eczématide achromiante et/ou des crevasses et/ou de la dermatite atopique et/ou de l'ichtyose et/ou les états de sécheresse de la peau et/ou des muqueuses accompagnant des pathologies cutanées et/ou mucosales telles que l'eczéma et/ou dans le traitement des états squameux, et/ou des démangeaisons associées aux peaux et/ou muqueuses sèches, et/ou dans le maintien ou le renfort de l'état d'hydratation des peaux et/ou muqueuses agressées, sensibilisées, altérées, intolérantes ou réactives.

Enfin, la présente invention concerne une méthode de soin cosmétique caractérisée en ce qu'elle comprend l'application sur au moins une zone concernée de la peau et/ou des muqueuses du visage ou du corps, en particulier des peaux et/ou muqueuses sensibles, séniles ou fragiles, de l'association selon la présente invention ou d'une composition cosmétique ou d'un mélange d'ingrédients cosmétiques ou pharmaceutiques comprenant à titre d'agent actif, l'association telle que définie précédemment, pour maintenir ou renforcer l'état d'hydratation de la peau, en particulier des peaux et/ou muqueuses sensibles, séniles ou fragiles , et/ou pour prévenir ou ralentir l'apparition des signes de sécheresse cutanée et/ou mucosale, en particulier pendant au moins 10 heures, avantageusement 24 heures, plus avantageusement jusqu'à 48 heures et/ou pour le traitement des états de sècheresse cutanée et/ou mucosale tels que les tiraillements associées aux peaux et/ou muqueuses sèches et/ou pour prévenir ou diminuer l'apparition des rides liées à une sécheresse cutanée, et/ou pour améliorer le confort des peaux et/ou muqueuses sèches, et/ou pour le traitement des peaux et/ou muqueuses d'aspect rugueux à la vue et/ou au toucher.

Elle décrit en outre une méthode de traitement et/ou de prévention et/ou de diminution de la survenance de gerçures et/ou des dartres ou eczématide achromiante et/ou des crevasses, et/ou de la dermatite atopique et/ou de l'ichtyose et/ou des états de sécheresse de la peau et/ou des muqueuses accompagnant des pathologies cutanées et/ou mucosales telles que l'eczéma et/ou de traitement des états squameux, et/ou des démangeaisons associées aux peaux et/ou muqueuses sèches, et/ou de maintien ou de renfort de l'état d'hydratation des peaux et/ou muqueuses agressées, sensibilisées, altérées, intolérantes ou réactives comprenant l'application, sur au moins une zone concernée de la peau et/ou des muqueuses du visage ou du corps d'un patient en ayant besoin, de l'association selon la présente invention ou d'une composition pharmaceutique ou d'un mélange d'ingrédients pharmaceutiques comprenant à titre d'agent actif, l'association telle que définie précédemment.

Ainsi, l'association selon la présente invention pouvant être utilisée avec des ingrédients actifs cosmétiques et/ou pharmaceutiques, en particulier dermatologiques pour en augmenter la teneur et la durée d'action au niveau de l'épiderme et/ou des muqueuses.

Ainsi, la composition selon la présente invention contenant cette association et optionnellement ces ingrédients actifs pourra être utilisée dans les applications suivantes, qui sont fonction des utilisations possibles de ces actifs :
produits de soin anti-oxydant, anti-inflammatoire, antirides / anti-âge, liftant / tenseur / lissant, pour l'éclat du teint, favorisant la réplication cellulaire, régulateur de séborrhée, matifiant, régulateur de la taille des pores de la peau, cicatrisant, hydratant, apaisant, amincissant, anti-UV / solaire, après-soleil /régénérant, produits de maquillage.

Enfin, la présente invention concerne une composition d'entretien, en particulier détergente, comprenant l'association selon la présente invention.

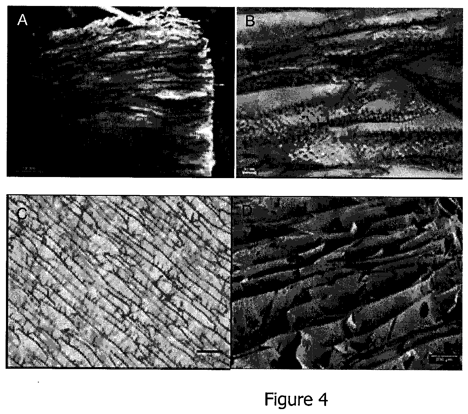

L'invention sera mieux comprise à la lecture de la description des figures et des exemples qui suivent.

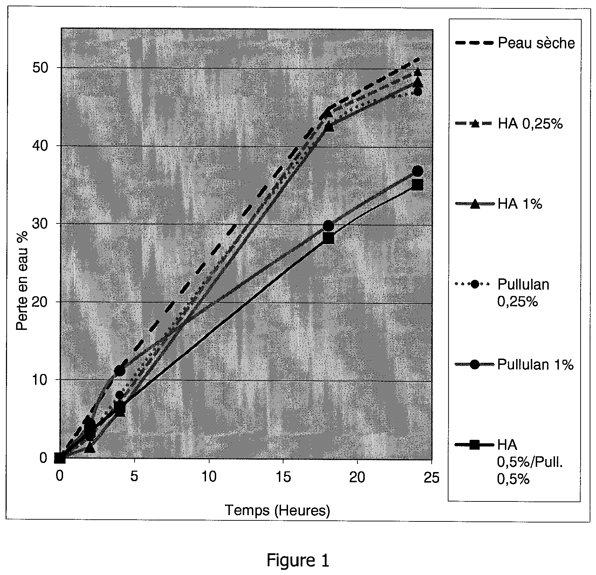

La **figure 1** représente la comparaison entre la mesure des pertes insensibles en fonction du temps obtenue avec une composition contenant l'association de 0,5 % en poids de pullulan et de 0,5 % en poids d'acide hyaluronique sous forme de hyaluronate de sodium (HA 0,5%/Pull. 0,5%) et la mesure des pertes insensibles en fonction du temps obtenue avec :
   - une composition contenant 0,25 % en poids de pullulan (Pullulan 0,25%),
   - une composition contenant 1 % en poids de pullulan (Pullulan 1%),
   - une composition contenant 0,25 % en poids d'acide hyaluronique, sous forme d'hyaluronate de sodium (HA 0,25%) et
   - une composition contenant 1 % en poids d'acide hyaluronique sous forme d'hyaluronate de sodium (HA 1%)
   par comparaison avec le contrôle (peaux synthétiques traitées avec la même quantité d'eau sans actif : peau sèche).

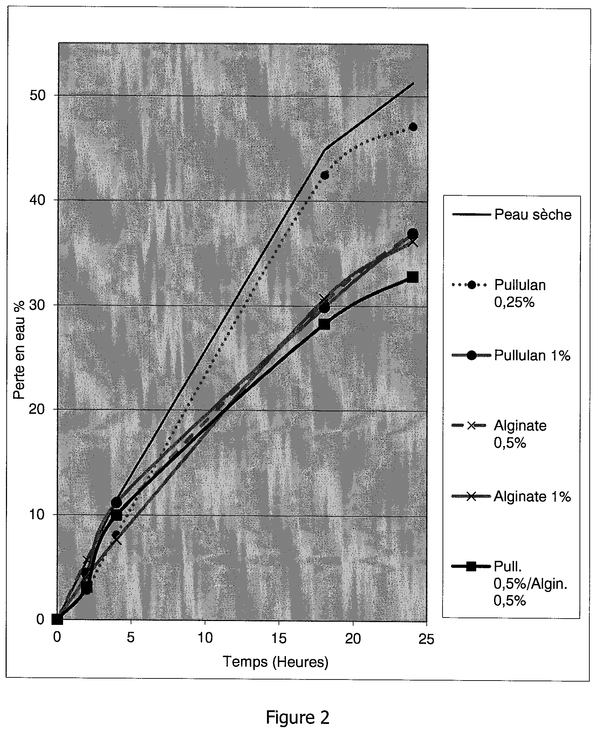

La **figure 2** représente la comparaison entre la mesure des pertes insensibles en fonction du temps obtenue avec une composition contenant l'association de 0,5 % en poids de pullulan et de 0,5 % en poids d'acide alginique sous forme d'alginate de sodium (Pull. 0,5%/Algin. 0,5%) et la mesure des pertes insensibles en fonction du temps obtenue avec :
   - une composition contenant 0,25 % en poids de pullulan (Pullulan 0,25%),
   - une composition contenant 1 % en poids de pullulan (Pullulan 1%),
   - une composition contenant 0,5 % en poids d'acide alginique sous forme d'alginate de sodium (Alginate 0,5%) et
   - une composition contenant 1 % en poids d'acide alginique sous forme d'alginate de sodium (Alginate 1%)
   par comparaison avec le contrôle (peaux synthétiques traitées avec la même quantité d'eau sans actif: peau sèche).

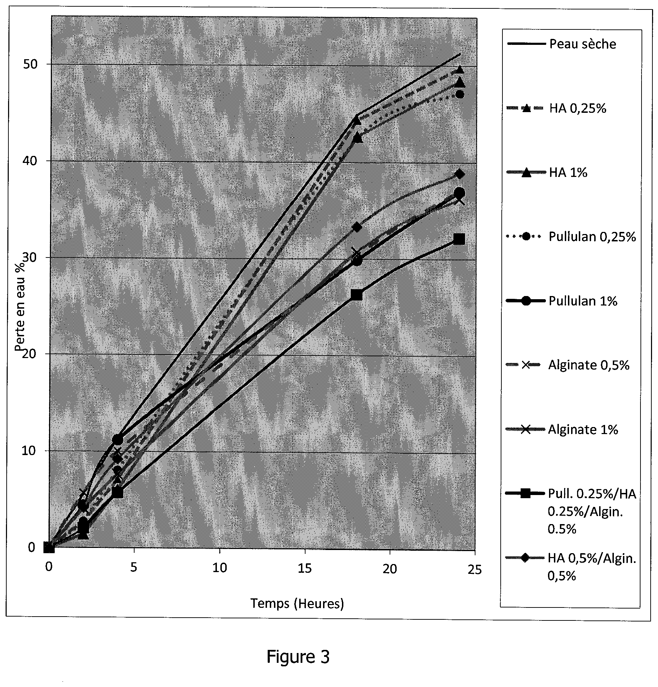

La **figure 3** représente la comparaison entre la mesure des pertes insensibles en fonction du temps obtenue avec une composition contenant l'association selon l'invention (0,25 % en poids de pullulan, 0,25 % en poids d'acide hyaluronique sous forme d'hyaluronate de sodium et 0,5 % en poids d'acide alginique sous forme d'alginate de sodium : Pull. 0,25%/HA 0,25%/Algin. 0,5%) et entre la mesure des pertes insensibles en fonction du temps obtenue avec :
   - une composition contenant 0,25 % en poids de pullulan (Pullulan 0,25%),
   - une composition contenant 1 % en poids de pullulan (Pullulan 1%),
   - une composition contenant 0,25 % en poids d'acide hyaluronique, sous forme d'hyaluronate de sodium (HA 0,25%) et
   - une composition contenant 1 % en poids d'acide hyaluronique sous forme d'hyaluronate de sodium (HA 1%)
   - une composition contenant 0,5 % en poids d'acide alginique sous forme d'alginate de sodium (Alginate 0,5%) et
   - une composition contenant 1 % en poids d'acide alginique sous forme d'alginate de sodium (Alginate 1%),
   - une composition contenant 0,5% en poids d'acide hyaluronique sous forme d'hyaluronate de sodium et 0,5% en poids d'acide alginique sous forme d'alginate de sodium (HA 0,5%/ Algin. 0,5%)
   par comparaison avec le contrôle (peaux synthétiques traitées avec la même quantité d'eau sans actif: peau sèche).
**La** **figure 4** représente la visualisation par microscopie du réseau de polymères formé par l'association selon l'invention lors de l'expérience décrite selon l'exemple 6 :
   **A.** Observation du réseau formé par l'association des 3 composés pullulan, hyaluronate de sodium et alginate de sodium par macrofluorescence.
   **B.** Observation selon A à plus grande échelle.
   **C.** Observation du réseau par microscopie optique (x10).
   **D.** Observation du réseau par microscopie électronique à transmission (x60).

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.
Ainsi, chaque exemple a une portée générale.

D'autre part, dans les exemples, et sauf indication contraire, la température est exprimée en degré Celsius et la pression est la pression atmosphérique.

### Exemple comparatif 1 : démonstration de l'effet synergique sur la diminution des pertes en eau de l'association du pullulan et de l'acide hyaluronique. (Figure 1)

### Principe :

Le test utilisé est un test in vitro mesurant la quantité d'eau évaporée en fonction du temps. Il s'inspire des tests classiques en cylindre ouvert mais a été effectué après séchage dans une étuve et non en atmosphère humide, ce qui accroit les pertes insensibles en eau et les miment dans des conditions beaucoup plus sévères que les tests classiques.

### Protocole :

Le protocole a été le suivant :
- Les différentes compositions à tester ont été préparées par mélange sous agitation planétaire des composants à différentes concentrations dans de l'eau permutée :
   - une composition contenant 0,25 % en poids de pullulan (Pullulan 0,25%),
   - une composition contenant 1 % en poids de pullulan (Pullulan 1%),
   - une composition contenant 0,25 % en poids d'acide hyaluronique, sous forme d'hyaluronate de sodium (HA 0,25%) et
   - une composition contenant 1 % en poids d'acide hyaluronique sous forme d'hyaluronate de sodium (HA 1%)
   - une composition contenant l'association de 0,5 % en poids de pullulan et de 0,5 % en poids d'acide hyaluronique sous forme de hyaluronate de sodium (HA 0,5%/Pull. 0,5%)
- Chaque composition a été appliquée en couche fine de 200 µm sur une peau synthétique (Vitroskin®). A titre de contrôle négatif une peau synthétique (Vitroskin®), a été traitée de la même manière avec une composition contenant uniquement cette quantité d'eau.
- La peau synthétique traitée a été séchée dans une étuve ventilée à 45 °C pendant 1 heure.
- Remplissage des cellules de forme cylindrique de 1cm de rayon et de 2 cm de hauteur avec de l'eau permutée (environ 6 cm3).
- Placer la peau traitée sur la cellule de telle sorte que la surface d'évaporation mesurée soit d'environ 3,14 cm².
- Déposer la cellule dans une étude ventilée à 45 °C.
- Peser la cellule et mesurer la perte en eau à t=0, 2 heures, 4 heures, 18 heures et 24 heures.

Chaque composition a été testée 3 fois et l'écart type a été calculé.

### Résultats :

Les compositions et les résultats obtenus sont rassemblés dans le tableau 1 ci-après:

**Tableau 1 : Pertes insensibles en eau mesurées (en % d'eau évaporé)**

| Composition | Temps (en heures) | 0 | 2 | 4 | 18 | 24 |
|---|---|---|---|---|---|---|
| -sans actif | Moyenne | 0 | 5,62 | 11,45 | 44,91 | 51,28 |
| | Ecart type | 0 | 1,43 | 1,97 | 8,32 | 8,56 |
| HA 0,25% | Moyenne | 0 | 2,73 | 7,13 | 44,35 | 49,72 |
| | Ecart type | 0 | 0,35 | 1,01 | 9,74 | 10,53 |
| HA 1% | Moyenne | 0 | 1,42 | 6,02 | 42,68 | 48,41 |
| | Ecart type | 0 | 0,20 | 0,37 | 1,89 | 2,43 |
| Pullulan 0,25% | Moyenne | 0 | 3,26 | 8,34 | 42,90 | 47,22 |
| | Ecart type | 0 | 0,74 | 0,49 | 4,06 | 5,75 |
| Pullulan 1% | Moyenne | 0 | 5,35 | 10,13 | 33,50 | 38,52 |
| | Ecart type | 0 | 0,74 | 0,25 | 0,54 | 0,85 |
| HA 0,5%/ Pull. 0,5% | Moyenne | 0 | 3,40 | 6,53 | 28,34 | 35,19 |
| | Ecart type | 0 | 1,46 | 2,74 | 4,60 | 4,15 |

### Conclusions :

Comme on peut le voir clairement sur la figure 1, les pertes insensibles en eau sont inférieures avec une composition contenant l'association de 0,5 % d'hyaluronate de sodium et de 0,5 % de pullulan à une teneur de 1 % en poids que pour une composition contenant 1 % en poids d'hyaluronate de sodium ou 1 % en poids de pullulan. Il y a donc bien synergie entre les composants. Cette synergie est particulièrement mise en évidence 5 heures après application, et très importante 10 heures après application de la composition et encore plus forte 18 heures après application de la composition et dure au moins 24 heures.

### Exemple comparatif 2 : démonstration de l'effet synergique sur la diminution des pertes en eau de l'association du pullulan et de l'acide alginique.(Figure 2)

### Principe et protocole :

Le protocole indiqué à l'exemple 1 est utilisé de la même manière avec les compositions aqueuses à tester suivantes :
- une composition contenant 0,25 % en poids de pullulan (Pullulan 0,25%),
- une composition contenant 1 % en poids de pullulan (Pullulan 1%),
- une composition contenant 0,5 % en poids d'acide alginique sous forme d'alginate de sodium (Alginate 0,5%) et
- une composition contenant 1 % en poids d'acide alginique sous forme d'alginate de sodium (Alginate 1%)
- une composition contenant l'association de 0,5 % en poids de pullulan et de 0,5 % en poids d'acide alginique sous forme d'alginate de sodium (Pull. 0,5%/Algin. 0,5%)
- une composition constituée d'eau sans actif

### Résultats :

Les compositions et les résultats obtenus sont rassemblés dans le tableau 2 ci-après :

**Tableau 2 : Pertes insensibles en eau mesurées (en % d'eau évaporé)**

| Composition | Temps (en heures) | 0 | 2 | 4 | 18 | 24 |
|---|---|---|---|---|---|---|
| sans actif | Moyenne | 0 | 5,62 | 11,45 | 44,91 | 51,28 |
| | Ecart type | 0 | 1,43 | 1,97 | 8,32 | 8,56 |
| Alginate 0,5% | Moyenne | 0 | 5,70 | 10,04 | 30,37 | 37,04 |
| | Ecart type | 0 | 0,25 | 0,47 | 3,50 | 4,87 |
| Alginate 1% | Moyenne | 0 | 4,08 | 7,64 | 30,71 | 36,20 |
| | Ecart type | 0 | 1,05 | 1,61 | 3,10 | 2,25 |
| Pullulan 0,25% | Moyenne | 0 | 3,26 | 8,34 | 42,90 | 47,22 |
| | Ecart type | 0 | 0,74 | 0,49 | 4,06 | 5,75 |
| Pullulan 1% | Moyenne | 0 | 5,35 | 10,13 | 33,50 | 38,52 |
| | Ecart type | 0 | 0,74 | 0,25 | 0,54 | 0,85 |
| Pull. 0,5%/ Algin. 0,5% | Moyenne | 0 | 3,10 | 9,98 | 28,30 | 32,84 |
| | Ecart type | 0 | 0,79 | 1,07 | 2,86 | 2,66 |

### Conclusions :

Comme on peut le voir clairement sur la figure 2, les pertes insensibles en eau sont inférieures pour une composition contenant l'association de 0,5 % d'alginate de sodium et de 0,5 % de pullulan à une teneur de 1 % en poids que pour une composition contenant 1 % en poids d'alginate de sodium ou 1 % en poids de pullulan. Il y a donc bien synergie entre les composants. Cette synergie est particulièrement mise en évidence 12 heures après application, et très importante 15 heures après application de la composition et encore plus forte 18 heures après application de la composition et dure au moins 24 heures.

### Exemple 3 : démonstration de l'effet synergique sur la diminution des pertes en eau de l'association selon la présente invention contenant du pullulan, de l'acide hyaluronique et de l'acide alginique.(Figure 3)

### Principe et protocole :

Le protocole indiqué à l'exemple 1 est utilisé de la même manière avec les compositions aqueuses à tester suivantes :
- une composition contenant 0,25 % en poids de pullulan (Pullulan 0,25%),
- une composition contenant 1 % en poids de pullulan (Pullulan 1%),
- une composition contenant 0,25 % en poids d'acide hyaluronique, sous forme d'hyaluronate de sodium (HA 0,25%) et
- une composition contenant 1 % en poids d'acide hyaluronique sous forme d'hyaluronate de sodium (HA 1%)
- une composition contenant 0,5 % en poids d'acide alginique sous forme d'alginate de sodium (Alginate 0,5%) et
- une composition contenant 1 % en poids d'acide alginique sous forme d'alginate de sodium (Alginate 1%)
- une composition contenant 0,5 % en poids d'acide hyaluronique sous forme d'hyaluronate de sodium et 0,5 % en poids d'acide alginique sous forme d'alginate de sodium (HA 0,5%/ Algin.0,5%)
- une composition contenant 0,25 % en poids de pullulan, 0,25 % en poids d'acide hyaluronique sous forme d'hyaluronate de sodium et 0,5 % en poids d'acide alginique sous forme d'alginate de sodium : Pull. 0,25%/HA 0,25%/Algin. 0,5%
- une composition constituée d'eau sans actif

### Résultats :

Les compositions et les résultats obtenus sont rassemblés dans le tableau 3 ci-après :

**Tableau 3 : Pertes insensibles en eau mesurées (en % d'eau évaporé)**

| composition | Temps (en heures) | 0 | 2 | 4 | 18 | 24 |
|---|---|---|---|---|---|---|
| sans actif | Moyenne | 0 | 5,62 | 11,45 | 44,91 | 51,28 |
| | Ecart type | 0 | 1,43 | 1,97 | 8,32 | 8,56 |
| HA 0,25% | Moyenne | 0 | 2,73 | 7,13 | 44,35 | 49,72 |
| | Ecart type | 0 | 0,35 | 1,01 | 9,74 | 10,53 |
| HA 1% | Moyenne | 0 | 1,42 | 6,02 | 42,68 | 48,41 |
| | Ecart type | 0 | 0,20 | 0,37 | 1,89 | 2,43 |
| Alginate 0,5% | Moyenne | 0 | 5,70 | 10,04 | 30,37 | 37,04 |
| | Ecart type | 0 | 0,25 | 0,47 | 3,50 | 4,87 |
| Alginate 1% | Moyenne | 0 | 4,08 | 7,64 | 30,71 | 36,20 |
| | Ecart type | 0 | 1,05 | 1,61 | 3,10 | 2,25 |
| Pullulan 0,25% | Moyenne | 0 | 3,26 | 8,34 | 42,90 | 47,22 |
| | Ecart type | 0 | 0,74 | 0,49 | 4,06 | 5,75 |
| Pullulan 1% | Moyenne | 0 | 5,35 | 10,13 | 33,50 | 38,52 |
| | Ecart type | 0 | 0,74 | 0,25 | 0,54 | 0,85 |
| HA 0,5%/ Algin. 0,5% | Moyenne | 0 | 4,17 | 9,21 | 33,32 | 38,90 |
| | Ecart type | 0 | 0,87 | 1,80 | 1,18 | 1,44 |
| Pull. 0,25%/ HA 0,25% / Algin. 0,5% | Moyenne | 0 | 2,05 | 5,73 | 26,36 | 32,17 |
| | Ecart type | 0 | 0,55 | 1,43 | 2,53 | 2,02 |

### Conclusions :

Comme on peut le voir clairement sur la figure 3, les pertes insensibles en eau sont plus réduites pour une composition contenant l'association selon la présente invention à une teneur de 1 % en poids sec (0,5 % d'acide alginique, 0,25 % d'acide hyaluronique et 0,5 % de pullulan) que pour une composition contenant 1 % en poids d'acide alginique ou 1 % en poids de pullulan ou 1 % en poids d'acide hyaluronique ou même 0,5 % en poids d'acide hyaluronique et 0,5 % en poids d'acide alginique. Il y a donc bien synergie entre les 3 composants. Cette synergie est particulièrement mise en évidence 4 heures après application de la composition et dure au moins 24 heures.

Cette synergie est plus importante que pour l'association simple de l'acide hyaluronique et du pullulan telle qu'exemplifiée à l'exemple 1 ou celle de l'acide alginique et du pullulan telle qu'exemplifiée à l'exemple 2.

Par ailleurs, l'association d'acide alginique et d'acide hyaluronique n'a aucun effet synergique sur les pertes insensibles en eau.

### Exemple 4 : Evaluation des propriétés d'une composition selon l'invention contenant l'association d'alginate de sodium, de hyaluronate de sodium et de pullulan sur la teneur en eau

### Principe :

Cette étude consiste à évaluer ex vivo par mesure de la conductivité diélectrique les propriétés d'hydratation de la couche cornée de l'association selon l'invention par comparaison avec des compositions qui ne comprennent pas cette association. Cette technique est basée sur le fait que plus la couche cornée est sèche, plus sa conduction électrique est faible, ceci du fait de la bipolarité des molécules d'eau et du champ électrique qu'elles induisent ainsi dans la couche cornée. Cette technique est classiquement utilisée pour la mesure du pouvoir d'hydratation des actifs topiques et a été décrite dans les publications suivantes : OBATA M, TAGAMI H: A rapid in vitro test to assess skin moisturizers. J. Soc. Cosmet. Chem., vol. 41, 235-242, 1990; OBATA M, TAGAMI H: Electrical détermination of water content and concentration profile in simulation model of in vivo stratum corneum. J. Invest. Dermatol., vol. 92, 854-859, 1989.

### Protocole :

Des biopsies de peau normale humaine d'abdomen issue de résidus opératoires sont prélevées et les feuillets épidermiques sont séparés du derme après chauffage à 60°C pendant deux minutes. La couche cornée « stratum corneum » est isolée par digestion enzymatique selon le protocole décrit dans la publication ROCHEFORT A, DROUOT P, LEDUC M, VASSALET R, AGACHE P: A new technique for evaluation of cosmetics effect on mechanical properties of stratum corneum and epidermis in vitro. Int. J. Cosm. Sci., vol. 8, 27-36, 1986 avec les adaptations ci-après mentionnées.

Le modèle de couche cornée est placé dans des chambres à humidité relative définie : 44% - saturée avec une solution de carbonate de potassium pendant 1 h Chaque modèle de couche cornée est testé dans les conditions suivantes :
- stratum corneum sans traitement, (TN1)
- stratum corneum traité avec un hydrogel placebo (TN2) et dont la composition A est la suivante :

| Nom commercial | Inci | Quantité (%) |
|---|---|---|
| eau | Water | Qsp 100% |
| Elestab™ 50J | Chorphenesin (and) methylparaben | 0,35 |
| Carbopol 980 | Carbomer | 0,60 |
| Eau | Water | 29,40 |
| NaOH 16% | Sodium hydroxide | 0,75 |

- stratum corneum traité avec l'hydrogel selon la composition A contenant 1% de la composition B ci-après, et dénommé alors TP1, ou contenant 3% de la composition B, dénommé alors TP2 :

**composition B :**

| Nom | Quantité % |
|---|---|
| eau | 55,58 |
| Pentylène glycol | 2 |
| Phosphate de potassium | 0,02 |
| Tréhalose | 10 |
| Sérine | 3 |
| Caprylyl glycol | 0,5 |
| Glycérine | 17,5 |
| Glyceryl polyacrylate | 0,3 |
| Urée | 10 |
| Phosphate disodium | 0,1 |

- stratum corneum traité avec un hydrogel de la composition A contenant 1% (M1) ou 2% (M2) ou 3% (M3) d'un mélange d'ingrédients cosmétiques selon l'exemple 7 décrit ci-après (ratio alginate/HA/Pullulan : 2/1/1).

Les modèles sont alors traités avec les préparations topiques par trois applications consécutives espacées de 30 minutes, Les doses appliquées sont de 1 mg / cm². Les mesures sont effectuées à 12 reprises pour chaque échantillon et pour chaque intervalle de temps.

La conductivité diélectrique a été mesurée avant traitement puis à 30 minutes, 1, 2, 4, 6, 24, et 48 heures après le traitement.

### Résultats :

Les résultats sont présentés dans le tableau 4.1. Les résultats présentés dans le tableau 4.2 s'ont exprimées en pourcentage d'augmentation de la conductivité diélectrique après traitement avec les compositions mentionnées contenant les produits testés par rapport à la mesure de conductivité diélectrique obtenue avec la couche cornée traitée avec la composition dite placebo (témoin négatif 2 :TN2).

**Tableau 4 : Mesures de la conductivité diélectrique après application des compositions à tester**

| Moy désigné la moyenne ; ET désigne Ecart Type | | | | | | | | | | |
|---|---|---|---|---|---|---|---|---|---|---|
| Composition testée | Temps en heure | 0 | 0.5 | 1 | 2 | 4 | 6 | 24 | 30 | 48 |
| TN1 | Moy | 14,3 | 15,0 | 15,6 | 15,3 | 16,1 | 17,1 | 15,8 | 15,6 | 16,0 |
| | ET | 0,8 | 0,9 | 0,8 | 0,9 | 1,3 | 1,2 | 1,0 | 1,0 | 1,3 |
| TN2 Placebo | Moy | 13,0 | 34,4 | 25,3 | 21,3 | 20,0 | 18,6 | 18,4 | 17,4 | 18,5 |
| | ET | 0,8 | 2,0 | 1,4 | 1,8 | 1,8 | 1,7 | 1,5 | 1,3 | 1,2 |
| TP1 | Moy | 15,1 | 39,0 | 28,4 | 31,3 | 27,2 | 25,0 | 25,0 | 24,3 | 26,4 |
| | ET | 1,0 | 1,7 | 1,1 | 1,9 | 1,3 | 1,3 | 1,2 | 1,5 | 1,2 |
| TP2 | Moy | 15,3 | 48,5 | 35,6 | 31,5 | 31,8 | 30,9 | 28,4 | 29,2 | 30,2 |
| | ET | 0,9 | 2,7 | 2,2 | 2,6 | 2,0 | 1,8 | 1,5 | 2,2 | 2,6 |
| M1 1% HA/Algin/pull | Moy | 14,5 | 46,3 | 33,2 | 33,1 | 31,6 | 31,9 | 30,6 | 27,7 | 25,6 |
| | ET | 0,6 | 2,5 | 1,5 | 1,2 | 2,5 | 2,0 | 2,1 | 1,7 | 2,2 |
| M2 2% HA/Algin/pull | Moy | 15,2 | 54,7 | 41,2 | 38,7 | 38,9 | 37,0 | 33,8 | 32,7 | 33,6 |
| | ET | 0,6 | 2,5 | 1,4 | 1,6 | 1,1 | 1,3 | 1,2 | 1,0 | 2,6 |
| M3 3% HA/Algin/pull | Moy | 15,9 | 67,3 | 53,2 | 47,6 | 45,8 | 44,1 | 39,4 | 40,6 | 38,0 |
| | ET | 0,9 | 3,4 | 2,8 | 3,1 | 3,2 | 2,6 | 2,4 | 2,2 | 2,9 |

**Tableau 4.2 :Pourcentage de variation par rapport au TN2 placebo - résultats en % en fonction du temps**

| Temps en heures Composition testée | 0 | 0,5 | 1 | 2 | 4 | 6 | 24 | 30 | 48 |
|---|---|---|---|---|---|---|---|---|---|
| TP2 : placebo+3% complexe hydratant | 17,3 | 41,0 | 41,0 | 48,2 | 59,2 | 66,1 | 54,1 | 67,8 | 63,5 |
| M3 : placebo+3% (complexe hydratant+Mélange HA/Pu/ALG (1/1/2)) | 22,0 | 95,5 | 110,5 | 124,2 | 129,2 | 136,7 | 113,7 | 133,3 | 105,9 |

### Conclusions:

La teneur en eau a été évaluée par mesure de conductivité diélectrique, laquelle est proportionnelle à l'état d'hydratation de la couche cornée.

Le contrôle négatif non traité démontre que, la teneur en eau dans la biopsie et dans les conditions du test a été stable au cours du temps. L'application des compositions a provoqué une augmentation de la conductivité diélectrique et donc de la teneur en eau de la couche cornée.

Les compositions M1, M2 et M3 contenant l'association selon l'invention démontrent une capacité à augmenter la teneur en eau de la peau et permette de conclure à un effet hydratant significatif versus la composition TN placebo.

La comparaison de la composition M1 contenant l'association selon l'invention avec la composition TP1, et des compositions M2 et M3 contenant l'association selon l'invention avec les compositions TP1 et TP2 démontre que l'association selon l'invention a une efficacité très supérieure et ce à tous les intervalles de temps jusqu'à 48heures ce qui traduit d'un effet immédiat et durable.

Cet exemple démontre que l'association selon l'invention augmente très efficacement et de manière durable la teneur en eau de la peau et/ou des muqueuses et donc constitue un très bon agent hydratant.

### Exemple 5 : Evaluation de la teneur en ingrédients actifs avec une composition contenant l'association selon l'invention

### Principe :

Cette étude ex vivo est effectuée sur cellules de Franz permettant d'étudier la diffusion de compositions testées au travers d'une biopsie de peau en fonction du temps.

### Protocole :

Des biopsies complètes de peau humaine normale d'abdomen ont été obtenues de résidus opératoires et insérées à l'interface des compartiments donneurs et receveurs des cellules de Franz. Les compositions testées ont été appliquées sur la face dite donneur/supérieur des cellules. Du tampon PBS a été utilisé comme fluide récepteur.

Les compositions testées ont été les suivantes :
- Témoin positif : composition B (contenant de la serine et de l'urée respectivement à 3% et 10%) (Composition témoin)
- Mélange d'ingrédient cosmétique selon l'exemple 7 ci-après (Composition selon l'invention)

Les cellules de Franz contenant les biopsies ont été incubées pendant 48 heures à 37°C avec les compositions à tester. Les compositions à tester ont ainsi été appliquées à la quantité de 300 microlitres sur la partie supérieure/donneur des cellules, ce qui correspond à l'application de 9 mg de sérine et 30 mg d'urée pour l'ensemble des compositions. Les biopsies ont été récupérées après 6, 10, 24 et 48 heures de traitement et la couche cornée, stratum corneum, a été retirée par huit décapage à l'aide d'adhésifs (technique de tape-strippings - D-Squame™, CuDerm Corp.). Les 8 adhésifs ont été réunis pour reconstituer la couche cornée et la sérine et l'urée contenues ont été extraits par tampon PBS et quantifiés par chromatographie en phase liquide à haute performance (HPLC) et quantifiées par étalon standard. La valeur obtenue avec le PBS a été soustrait à chaque mesure. L'expérience a été menée en quintuplicata (n=5)

### Résultats :

La significativité a été déterminée par test de Student dit t-test avec un seuil fixé à 5% (p<0.05)

Les résultats pour la sérine sont présentés dans le tableau 5. 1 et pour l'urée dans le tableau 5.2.

**Tableau 5.1 : Stockage de la sérine dans le stratum corneum**

| Compositions testées | Temps en heures | 6 | 10 | 24 | 48 |
|---|---|---|---|---|---|
| Composition témoin | Moyenne | 4,53 | 6,30 | 18,66 | 21,34 |
| | Ecart Type | 3,99 | 4,23 | 11,35 | 5,35 |
| Composition selon invention | Moyenne | 21,54 | 69,78 | 79,17 | 81,57 |
| | Ecart Type | 4,75 | 16,89 | 59,68 | 10,51 |
| Comparaison « composition selon invention vs composition témoin (en %) » | | +353 | +313 | +324 | +282 |

**Tableau 5.2 : Stockage de l'urée dans le stratum corneum**

| Compositions testées | Temps en heures | 6 | 10 | 24 | 48 |
|---|---|---|---|---|---|
| Composition témoin | Moyenne | 49,69 | 46,40 | 89,82 | 104,11 |
| | Ecart Type | 10,79 | 10,92 | 35,05 | 34,14 |
| Composition selon invention | Moyenne | 125,12 | 228,41 | 269,02 | 272,74 |
| | Ecart Type | 29,74 | 56,65 | 130,33 | 92,87 |
| Comparaison « composition selon invention vs composition témoin (en %) » | | +152 | +392 | +200 | +162 |

### Conclusions :

Les résultats démontrent que la sérine contenue dans la composition selon l'invention est beaucoup mieux retenue dans la couche cornée après 6 heures mais également après 10, 24 et 48 heures que lorsqu'elle est dans une composition témoin sans polymère.

De même, l'urée lorsqu'appliquée avec la composition selon l'invention est mieux retenue et conserve dans la couche cornée à 6 heures mais également après 10, 24 et 48 heures.

Les résultats de cette étude ex vivo démontrent que lorsqu'elles sont appliquées sous la forme d'une composition selon l'invention la sérine et l'urée sont mieux retenues dans la couche cornée et leur teneur dans celle-ci en est augmentée. L'association selon l'invention augmente donc la teneur des actifs dans le stratum corneum.

### Exemple 6 : Visualisation par microscopie du réseau de polymères formé par l'association selon l'invention

### Protocole :

Une solution aqueuse contenant 0,25 % de pullulan, 0,25 % de hyaluronate de sodium et 0,5 % d'alginate de sodium en poids, selon un ratio 1/1/2 est homogénéisée puis lyophilisée. L'association ainsi obtenue sous forme de réseau déshydraté est ensuite finement découpée (5x10cm) pour une observation par macrofluorescence ou par microscopie électronique à transmission.

Pour l'observation par microscopie optique, 1 mL de la solution aqueuse contenant 0,25 % de pullulan, 0,25 % de hyaluronate de sodium et 0,5 % d'alginate de sodium en poids, selon un ration 1/1/2 est prélevée avant lyophilisation puis laissé séchée 16 heures_à température ambiante (20 °C) avant observation.

### Résultats :

Les résultats sont présentés à la Figure 4.

### Conclusions :

Dans le ratio 1/1/2, l'association selon l'invention, du pullulan, de l'acide hyaluronique et de l'acide alginique forme un réseau moléculaire pluristratifié comme observé ci-dessus par microscopie. En particulier, l'observation par microscopie électronique à transmission (D) met clairement en évidence la structure en feuillet du réseau formé. La formation de ce réseau moléculaire qui induit un effet retard sur la diffusion percutanée du ou des ingrédients actifs contenus dans les compositions selon l'invention et ainsi augmente et prolonge au cours du temps la teneur en ingrédient(s) actif(s), comme démontré dans l'exemple 5 précédent.

### Exemple 7 : Mélange d'ingrédients cosmétiques ou pharmaceutiques selon l'invention contenant l'association d'alginate de sodium, de hyaluronate de sodium et de pullulan

Un mélange d'ingrédients cosmétiques ou pharmaceutiques ayant la formulation ci-après en pourcentage en poids est préparé.

| Dénomination | Quantité en % |
|---|---|
| Alginate de sodium | 0,5 |
| Pentylène glycol | 2 |
| Phosphate de potassium | 0,02 |
| Tréhalose | 10 |
| Sérine | 3 |
| Hyaluronate de sodium | 0,25 |
| Caprylyl glycol | 0,5 |
| Pullulan | 0,25 |
| Glycérine | 17,5 |
| Polyacrylate de glycérine | 0,3 |
| Urée | 10 |
| Disodium de phosphate | 0,1 |
| Eau | QSP |

Le procédé de préparation est le suivant : dans de l'eau tamponnée à pH 6 avec un mélange de sels phosphate disodium/ phosphate de potassium, on ajoute le hyaluronate de sodium et le pullulan. Une fois que le mélange de polymères est obtenu on ajoute l'urée, le tréhalose, la sérine et la glycérine. Après complète solubilisation, l'alginate de sodium, le pentylène glycol, le caprylyl glycol et le gel à base de polyacrylate de glycérile sont ajoutés de façon à stabiliser le produit.

### Exemple 8 : Composition selon l'invention sous la forme d'une Lotion hydratante pour le corps et/ou le visage:

| Phase | Dénomination | Quantité (% en poids total) |
|---|---|---|
| A | Eau | 69.90 |
| A | Disodium EDTA | 0.05 |
| A | Gomme xanthane | 0.20 |
| B | Steareth -2 | 2.00 |
| B | Stereth-21 | 2.50 |
| B | Alcool cetearyl | 1.00 |
| B | Caprylate de propylheptyl | 15.00 |
| C | Mélange d'ingrédients cosmétiques selon l'exemple 7 comprenant l'association selon l'invention | 3.00 |
| D | Eau | 1.00 |
| D | Hydroxide de sodium (30% en solution) | 0.10 |
| E | Mélange de phenoxyéthanol, chlorphenesin, acide benzoique, butylène glycol, acide sorbique (Germazide ™ PBS) | 1.25 |
| F | Mélange de polyacrylate-X, d'isohexadecane et de polysorbate 60 (Sepigel™ SMS 60) | 4.00 |

La lotion est préparée par les méthodes usuelles dans le domaine bien connu de l'homme du métier, en mélangeant les 6 phases.

### Exemple 9 : Evaluation in vivo de l'effet hydratant d'une composition selon l'invention sous la forme d'une cosmétique ou pharmaceutique :

### Principe :

Cette étude permet de déterminer in vivo l'hydratation immédiate procurée de l'association selon l'invention par un cornéomètre après une seule application.

### Protocole :

L'étude a été conduite sur 22 femmes âgées de 18 à 65 ans.

Une légère déstructuration de l'épiderme sur la zone de tests a été effectuée avant le test consistant en un traitement préalable de 3 jours de nettoyage au savon, deux fois par jour pour mimer une peau dite « très sèche ». La composition selon l'exemple 8 sans le mélange d'ingrédients cosmétiques selon l'invention dite composition Placebo ou avec le mélange d'ingrédients cosmétiques selon l'invention dite composition selon l'invention a été appliquée sur l'avant-bras en monoapplication (quantité approximative de 40microlitre sur 3x3cm²).

L'hydratation a été mesurée à 30 minutes, 4, 8, 24 et 48 heures par cornéomètre après application et en triplicate.

### Résultats :

Les résultats sont exprimés en pourcentage de variation de la valeur mesurée par rapport à la valeur initiale mesurée avant le test, ce qui traduit le pourcentage d'amélioration de l'hydratation.

**Tableau 8 :**

| | | | | | |
|---|---|---|---|---|---|
| Temps après application (heure) ----- Composition testée | 0.5 | 4 | 8 | 24 | 48 |
| Composition Placebo | 58 | 14 | 20 | 24 | 28 |
| Composition selon l'invention | 73 | 24 | 30 | 32 | 33 |

| | | | | | |
|---|---|---|---|---|---|
| La différence observée entre la composition Placebo et la composition selon l'invention est toujours significative d'après le t- test (p<0.05) | | | | | |

### Conclusion

La composition selon l'invention a amélioré la teneur en eau de la peau de manière significative comparée à la composition Placebo. Cette amélioration est immédiate, dès 30 minutes et durable dans le temps.

L'expérience fut renouvelée sur hémivisage consistant en l'application de la composition selon l'invention ou de la composition Placebo sur le visage par application répétée deux fois par jour pendant 20 jour et le matin du 21^{ème} jour sur une population de femmes similaires a également montré que, au 23^{ème} et 26^{ème} jour, soit deux jours ou cinq jours après l'arrêt des applications, la teneur en eau de la peau des femmes ayant utilisé la composition Placebo et mesurée avec le cornéomètre était revenue à son état initial alors que le visage les femmes ayant utilisé la composition selon l'invention présentait toujours une amélioration significative de la teneur en eau de leur peau même après arrêt du traitement (+14% au 23^{ème} jour soit 2 jours après l'arrêt du traitement et + 12% au 26^{ème} jour) ce qui traduit la rémanence de cet effet.

## Revendications

1. Association du pullulan ou d'un de ses dérivés estérifiés ou d'un de ses dérivés organominéraux à base de silicium avec un mélange de polysaccharides consistant en l'acide hyaluronique ou l'un de ses sels ou dérivés estérifiés ou dérivés organominéraux à base de silicium et l'acide alginique ou l'un de ses sels ou dérivés estérifiés ou dérivés organominéraux à base de silicium, le ratio en poids pullulan ou dérivés estérifiés ou dérivés organominéraux à base de silicium / acide hyaluronique, sels ou dérivés estérifiés ou dérivés organominéraux à base de silicium / acide alginique, sels ou dérivés estérifiés ou dérivés organominéraux à base de silicium étant compris dans la gamme de 1/1/1 à 1/10/10.

2. Association selon la revendication 1 **caractérisée en ce que** le pullulan a un poids moléculaire inférieur à 500 kDa.

3. Association selon l'une quelconque des revendications 1 ou 2 **caractérisée en ce que** l'acide hyaluronique, ses sels ou dérivés estérifiés ont un poids moléculaire supérieur à 20 kDa, avantageusement compris entre 50 et 800 kDa.

4. Association selon l'une quelconque des revendications 1 à 3 **caractérisée en ce qu'**elle comprend un sel de l'acide hyaluronique, avantageusement le hyaluronate de sodium.

5. Association selon l'une quelconque des revendications 1 à 4 **caractérisée en ce qu'**elle comprend un sel de l'acide alginique, avantageusement l'alginate de sodium.

6. Association selon l'une quelconque des revendications 1 à 5 **caractérisée en ce que** le ratio en poids de pullulan ou dérivés estérifiés ou dérivés organominéraux à base de silicium / acide hyaluronique, sels ou dérivés estérifiés ou dérivés organominéraux à base de silicium / acide alginique, sels ou dérivés estérifiés ou dérivés organominéraux à base de silicium est compris dans la gamme 1/1/1 à 1/2/5.

7. Association selon l'une des revendications 1 à 6, **caractérisée en ce que** le ratio en poids de pullulan ou dérivés estérifiés ou dérivés organominéraux à base de silicium /acide alginique, sels ou dérivés estérifiés ou dérivés organominéraux à base de silicium est compris dans la gamme 1/1 à 1/2.

8. Association selon l'un des revendications 1 à 7, **caractérisée en ce que** le ratio en poids pullulan ou dérivés estérifiés ou dérivés organominéraux à base de silicium / acide hyaluronique, sels ou dérivés estérifiés ou dérivés organominéraux à base de silicium / acide alginique, sels ou dérivés estérifiés ou dérivés organominéraux à base de silicium est de 1/1/2.

9. Utilisation de l'association selon l'une quelconque des revendications 1 à 8 pour diminuer les pertes insensibles en eau et/ou pour augmenter la teneur en eau de la peau et/ou des muqueuses et/ou pour augmenter et/ou prolonger la teneur en ingrédients actifs cosmétiques et/ou pharmaceutiques dans la peau et/ou les muqueuses et/ou la maintenir dans le temps et/ou comme agent hydratant de la peau et/ou des muqueuses.

10. Mélange d'ingrédients cosmétiques ou pharmaceutiques destiné à être incorporé dans une composition cosmétique ou pharmaceutique, ledit mélange comprenant l'association selon l'une quelconque des revendications 1 à 8 en une teneur comprise entre 0,1 et 5% en poids de matière sèche par rapport au poids total du mélange d'ingrédients, avantageusement entre 1 et 3% en poids et un véhicule cosmétique ou pharmaceutique approprié.

11. Mélange d'ingrédients cosmétiques ou pharmaceutiques selon la revendication 10 **caractérisé en ce qu'**il comprend
- le pullulan ou d'un de ses dérivés cosmétiquement ou pharmaceutiquement acceptable en une teneur comprise entre 0,01 et 3% en poids de matière sèche par rapport au poids total du mélange d'ingrédients cosmétiques ou pharmaceutiques, avantageusement entre 0,1 et 1% en poids et
- l'acide hyaluronique ou un de ses sels ou dérivés cosmétiquement ou pharmaceutiquement acceptable en une teneur comprise entre 0,01 et 3% en poids de matière sèche par rapport au poids total en matière sèche du mélange d'ingrédients cosmétiques ou pharmaceutiques avantageusement entre 0,1 et 1% en poids et/ou
- l'acide alginique ou un de ses sels ou dérivés cosmétiquement ou pharmaceutiquement acceptable en une teneur comprise entre 0,01 et 3% en poids de matière sèche par rapport au poids total du mélange d'ingrédients cosmétiques ou pharmaceutiques.

12. Mélange d'ingrédients cosmétiques ou pharmaceutiques selon l'une quelconque des revendications 10 ou 11 **caractérisé en ce qu'**il comprend en outre un autre agent hydratant, en particulier choisi parmi le tréhalose, la sérine, l'urée et leurs mélanges.

13. Mélange d'ingrédients cosmétiques ou pharmaceutiques selon l'une quelconque des revendications 10 à 12 **caractérisé en ce qu'**il contient l'acide alginique ou un de ses sels ou dérivés cosmétiquement ou pharmaceutiquement acceptable en une teneur comprise entre 0,1 et 2% en poids de matière sèche par rapport au poids total du mélange d'ingrédients cosmétiques ou pharmaceutiques.

14. Composition cosmétique ou pharmaceutique destinée à une administration par voie topique comprenant l'association selon l'une quelconque des revendications 1 à 8 en une teneur comprise entre 0,0001% et 20% en poids de matière sèche par rapport au poids total de la composition, avantageusement entre 0,01 et 5% en poids, et un véhicule cosmétique ou pharmaceutique approprié.

15. Composition selon la revendication 14 **caractérisée en ce qu'**elle comprend
- le pullulan ou un de ses dérivés estérifiés ou dérivés organominéraux à base de silicium cosmétiquement ou pharmaceutiquement acceptable en une teneur comprise entre 0,0001 et 10% en poids de matière sèche par rapport au poids total de la composition avantageusement entre 0,005 et 0,1% en poids et
- l'acide hyaluronique ou un de ses sels ou dérivés estérifiés ou dérivés organominéraux à base de silicium cosmétiquement ou pharmaceutiquement acceptable en une teneur comprise entre 0,0001 et 10% en poids de matière sèche par rapport au poids total en matière sèche de la composition, avantageusement entre 0,005 et 0,1% en poids et/ou
- l'acide alginique ou un de ses sels ou dérivés estérifiés ou dérivés organominéraux à base de silicium cosmétiquement ou pharmaceutiquement acceptable en une teneur comprise entre 0,0001 et 10% en poids de matière sèche par rapport au poids total de la composition, avantageusement entre 0,007 et 0,2% en poids.

16. Composition selon l'une quelconque des revendications 14 ou 15 **caractérisée en ce qu'**elle contient l'acide alginique ou un de ses sels ou dérivés estérifiés ou dérivés organominéraux à base de silicium cosmétiquement ou pharmaceutiquement acceptable en une teneur comprise entre 0,001 et 1% en poids de matière sèche par rapport au poids total de la composition.

17. Composition selon la revendication 16 **caractérisée en ce qu'**elle contient l'acide alginique ou un de ses sels ou dérivés estérifiés ou dérivés organominéraux à base de silicium cosmétiquement ou pharmaceutiquement acceptable en une teneur comprise entre 0,007 et 0,2% en poids de matière sèche par rapport au poids total de la composition.

18. Composition selon l'une quelconque des revendications 14 à 17 **caractérisée en ce qu'**elle se présente sous la forme d'un sérum, d'une lotion, d'une crème, d'un lait, d'une pommade, d'une pâte, d'une mousse, d'une émulsion, d'un hydrogel, d'un gel douche, d'un masque, d'un stick d'un patch, ou de poudres de maquillage, avantageusement d'une crème ou d'une lotion.

19. Composition selon l'une quelconque des revendications 14 à 18 **caractérisée en ce qu'**elle contient l'association du pullulan, ou dérivés estérifiés ou dérivés organominéraux à base de silicium / acide hyaluronique, sels ou dérivés estérifiés ou dérivés organominéraux à base de silicium / acide alginique, sels ou dérivés estérifiés ou dérivés organominéraux à base de silicium dans un ratio compris dans la gamme 1/1/1 à 1/2/5.

20. Utilisation de la composition cosmétique selon l'une quelconque des revendications 14 à 19 pour maintenir ou renforcer l'état d'hydratation de la peau et/ou muqueuse, en particulier des peaux et/ou muqueuses séniles, et/ou pour prévenir ou ralentir l'apparition des signes de sécheresse cutanée et/ou mucosale, en particulier pendant au moins 10 heures, et/ou pour le traitement des états de sècheresse cutanée et/ou mucosale que sont les tiraillements associées aux peaux et/ou muqueuses sèches et/ou pour prévenir ou diminuer l'apparition des rides liées à une sécheresse cutanée, et/ou pour améliorer le confort des peaux et/ou muqueuses sèches, et/ou pour le traitement des peaux et/ou muqueuses d'aspect rugueux à la vue et/ou au toucher.

21. Composition pharmaceutique selon l'une quelconque des revendications 14 à 19 pour son utilisation dans le traitement et/ou la prévention des gerçures et/ou des dartres ou eczématide achromiante et/ou des crevasses et/ou de la dermatite atopique et/ou de l'ichtyose et/ou des états de sécheresse de la peau ou des muqueuses accompagnant des pathologies cutanées et/ou mucosales telles que l'eczéma et/ou dans le maintien ou le renfort de l'état d'hydratation des peaux et/ou muqueuses agressées, sensibles, fragiles, sensibilisées, altérées, intolérantes ou réactives et/ou dans le traitement des états squameux et/ou des démangeaisons associées aux peaux et/ou muqueuses sèches.

22. Composition d'entretien, en particulier détergente, comprenant l'association selon l'une quelconque des revendications 1 à 8.

## Patentansprüche

1. Verbindung von Pullulan oder einem seiner veresterten Derivate oder einem seiner organomineralischen Derivate auf Siliciumbasis mit einem Gemisch von Polysacchariden, das aus Hyaluronsäure oder einem ihrer Salze oder veresterten Derivate oder organomineralischen Derivate auf Siliciumbasis und Alginsäure oder einem ihrer Salze oder veresterten Derivate oder organomineralischen Derivate auf Siliciumbasis besteht, wobei das Gewichtsverhältnis von Pullulan oder veresterten Derivaten oder organomineralischen Derivaten auf Siliciumbasis : Hyaluronsäure, Salzen oder veresterten Derivaten oder organomineralischen Derivaten auf Siliciumbasis : Alginsäure, Salzen oder veresterten Derivaten oder organomineralischen Derivaten auf Siliciumbasis im Bereich von 1:1:1 bis 1:10:10 liegt.

2. Verbindung nach Anspruch 1, **dadurch gekennzeichnet, dass** das Pullulan eine Molmasse von weniger als 500 kDa aufweist.

3. Verbindung nach einem der Ansprüche 1 oder 2, **dadurch gekennzeichnet, dass** die Hyaluronsäure, ihre Salze oder veresterten Derivate eine Molmasse von mehr als 20 kDa, vorteilhafterweise zwischen 50 und 800 kDa aufweisen.

4. Verbindung nach einem der Ansprüche 1 bis 3, **dadurch gekennzeichnet, dass** sie ein Salz von Hyaluronsäure, vorteilhafterweise Natriumhyaluronat umfasst.

5. Verbindung nach einem der Ansprüche 1 bis 4, **dadurch gekennzeichnet, dass** sie ein Salz von Alginsäure, vorteilhafterweise Natriumalginat umfasst.

6. Verbindung nach einem der Ansprüche 1 bis 5, **dadurch gekennzeichnet, dass** das Gewichtsverhältnis von Pullulan oder veresterten Derivaten oder organomineralischen Derivaten auf Siliciumbasis : Hyaluronsäure, Salzen oder veresterten Derivaten oder organomineralischen Derivaten auf Siliciumbasis : Alginsäure, Salzen oder veresterten Derivaten oder organomineralischen Derivaten auf Siliciumbasis im Bereich von 1:1:1 bis 1:2:5 liegt.

7. Verbindung nach einem der Ansprüche 1 bis 6, **dadurch gekennzeichnet, dass** das Gewichtsverhältnis von Pullulan oder veresterten Derivaten oder organomineralischen Derivaten auf Siliciumbasis : Alginsäure, Salzen oder veresterten Derivaten oder organomineralischen Derivaten auf Siliciumbasis im Bereich von 1:1 bis 1:2 liegt.

8. Verbindung nach einem der Ansprüche 1 bis 7, **dadurch gekennzeichnet, dass** das Gewichtsverhältnis von Pullulan oder veresterten Derivaten oder organomineralischen Derivaten auf Siliciumbasis : Hyaluronsäure, Salzen oder veresterten Derivaten oder organomineralischen Derivaten auf Siliciumbasis : Alginsäure, Salzen oder veresterten Derivaten oder organomineralischen Derivaten auf Siliciumbasis 1:1:2 beträgt.

9. Verwendung der Verbindung nach einem der Ansprüche 1 bis 8 zur Verringerung von insensiblen Wasserverlusten und/oder zur Erhöhung des Wassergehalts der Haut und/oder von Schleimhäuten und/oder zur Erhöhung und/oder Verlängerung des Gehalts an kosmetischen und/oder pharmazeutischen Wirkbestandteilen in der Haut und/oder den Schleimhäuten und/oder Aufrechterhalten dieses über die Zeit und/oder als Feuchthaltemittel für die Haut und/oder Schleimhäute.

10. Gemisch von kosmetischen oder pharmazeutischen Bestandteilen, die dazu vorgesehen sind, in eine kosmetische oder pharmazeutische Zusammensetzung integriert zu werden, wobei das Gemisch die Verbindung nach einem der Ansprüche 1 bis 8 in einem Gehalt zwischen 0,1 und 5 Gew.-% in Trockenmasse, bezogen auf das Gesamtgewicht des Gemischs von Bestandteilen, vorteilhafterweise zwischen 1 und 3 Gew.-%, und ein kosmetisch oder pharmazeutisch geeignetes Vehikel umfasst.

11. Gemisch von kosmetischen oder pharmazeutischen Bestandteilen nach Anspruch 10, **dadurch gekennzeichnet, dass** es umfasst:
- das Pullulan oder eines seiner kosmetisch oder pharmazeutisch annehmbaren Derivate in einem Gehalt zwischen 0,01 und 3 Gew.-% in Trockenmasse, bezogen auf das Gesamtgewicht des Gemischs von kosmetischen oder pharmazeutischen Bestandteilen, vorteilhafterweise zwischen 0,1 und 1 Gew.-%, und
- die Hyaluronsäure oder eines ihrer kosmetisch oder pharmazeutisch annehmbaren Salze oder Derivate in einem Gehalt zwischen 0,01 und 3 Gew.-% in Trockenmasse, bezogen auf das Gesamtgewicht des Gemischs von kosmetischen oder pharmazeutischen Bestandteilen in Trockenmasse, vorteilhafterweise zwischen 0,1 und 1 Gew.-%, und/oder
- die Alginsäure oder eines ihrer kosmetisch oder pharmazeutisch annehmbaren Salze oder Derivate in einem Gehalt zwischen 0,01 und 3 Gew.-% in Trockenmasse, bezogen auf das Gesamtgewicht des Gemischs von kosmetischen oder pharmazeutischen Bestandteilen.

12. Gemisch von kosmetischen oder pharmazeutischen Bestandteilen nach einem der Ansprüche 10 oder 11, **dadurch gekennzeichnet, dass** es ferner ein anderes Feuchthaltemittel umfasst, das insbesondere aus Trehalose, Serin, Harnstoff und deren Gemischen ausgewählt ist.

13. Gemisch von kosmetischen oder pharmazeutischen Bestandteilen nach einem der Ansprüche 10 bis 12, **dadurch gekennzeichnet, dass** es die Alginsäure oder eines ihrer kosmetisch oder pharmazeutisch annehmbaren Salze oder Derivate in einem Gehalt zwischen 0,1 und 2 Gew.-% in Trockenmasse, bezogen auf das Gesamtgewicht des Gemischs von kosmetischen oder pharmazeutischen Bestandteilen, enthält.

14. Kosmetische oder pharmazeutische Zusammensetzung, die für eine Verabreichung auf topischem Weg vorgesehen ist und welche die Verbindung nach einem der Ansprüche 1 bis 8 in einem Gehalt zwischen 0,0001 Gew.-% und 20 Gew.-% in Trockenmasse, bezogen auf das Gesamtgewicht der Zusammensetzung, vorteilhafterweise zwischen 0,01 und 5 Gew.-%, und ein kosmetisch oder pharmazeutisch geeignetes Vehikel umfasst.

15. Zusammensetzung nach Anspruch 14, **dadurch gekennzeichnet, dass** sie umfasst:
- das Pullulan oder eines seiner kosmetisch oder pharmazeutisch annehmbaren veresterten Derivate oder organomineralischen Derivate auf Siliciumbasis in einem Gehalt zwischen 0,0001 und 10 Gew.-% in Trockenmasse, bezogen auf das Gesamtgewicht der Zusammensetzung, vorteilhafterweise zwischen 0,005 und 0,1 Gew.-%, und
- die Hyaluronsäure oder eines ihrer kosmetisch oder pharmazeutisch annehmbaren Salze oder veresterten Derivate oder organomineralischen Derivate auf Siliciumbasis in einem Gehalt zwischen 0,0001 und 10 Gew.-% in Trockenmasse, bezogen auf das Gesamtgewicht der Zusammensetzung in Trockenmasse, vorteilhafterweise zwischen 0,005 und 0,1 Gew.-%, und/oder
- die Alginsäure oder eines ihrer kosmetisch oder pharmazeutisch annehmbaren Salze oder veresterten Derivate oder organomineralischen Derivate auf Siliciumbasis in einem Gehalt zwischen 0,0001 und 10 Gew.-% in Trockenmasse, bezogen auf das Gesamtgewicht der Zusammensetzung, vorteilhafterweise zwischen 0,007 und 0,2 Gew.-%.

16. Zusammensetzung nach einem der Ansprüche 14 oder 15, **dadurch gekennzeichnet, dass** sie die Alginsäure oder eines ihrer kosmetisch oder pharmazeutisch annehmbaren Salze oder veresterten Derivate oder organomineralischen Derivate auf Siliciumbasis in einem Gehalt zwischen 0,001 und 1 Gew.-% in Trockenmasse, bezogen auf das Gesamtgewicht der Zusammensetzung, enthält.

17. Zusammensetzung nach Anspruch 16, **dadurch gekennzeichnet, dass** sie die Alginsäure oder eines ihrer kosmetisch oder pharmazeutisch annehmbaren Salze oder veresterten Derivate oder organomineralischen Derivate auf Siliciumbasis in einem Gehalt zwischen 0,007 und 0,2 Gew.-% in Trockenmasse, bezogen auf das Gesamtgewicht der Zusammensetzung, enthält.

18. Zusammensetzung nach einem der Ansprüche 14 bis 17, **dadurch gekennzeichnet, dass** sie in der Form eines Serums, einer Lotion, einer Creme, einer Milch, einer Salbe, einer Paste, eines Schaums, einer Emulsion, eines Hydrogels, eines Duschgels, einer Maske, eines Stifts, eines Pflasters oder von Schminkpudern, vorteilhafterweise einer Creme oder einer Lotion dargereicht wird.

19. Zusammensetzung nach einem der Ansprüche 14 bis 18, **dadurch gekennzeichnet, dass** sie die Verbindung von Pullulan oder veresterten Derivaten oder organomineralischen Derivaten auf Siliciumbasis : Hyaluronsäure, Salzen oder veresterten Derivaten oder organomineralischen Derivaten auf Siliciumbasis : Alginsäure, Salzen oder veresterten Derivaten oder organomineralischen Derivaten auf Siliciumbasis in einem Verhältnis im Bereich von 1:1:1 bis 1:2:5 enthält.

20. Verwendung der kosmetischen Zusammensetzung nach einem der Ansprüche 14 bis 19 zur Aufrechterhaltung oder Verstärkung des Hydratationszustands der Haut und/oder von Schleimhaut, insbesondere von alter Haut und/oder alten Schleimhäuten, und/oder zur Verhinderung oder Verzögerung des Auftretens von Anzeichen von Haut- und/oder Schleimhauttrockenheit, insbesondere für mindestens 10 Stunden, und/oder zur Behandlung von Haut- und/oder Schleimhauttrockenheitszuständen, wie mit trockener Haut und/oder trockenen Schleimhäuten zusammenhängende Spannungsgefühle, und/oder zur Verhinderung oder Verringerung des Auftretens von Falten, die mit einer Hauttrockenheit in Verbindung stehen, und/oder zur Verbesserung des Komfortgefühls von trockener Haut und/oder trockenen Schleimhäuten, und/oder zur Behandlung von Haut und/oder Schleimhäuten, die rau aussehen und/oder sich rau anfühlen.

21. Pharmazeutische Zusammensetzung nach einem der Ansprüche 14 bis 19 zu deren Verwendung bei der Behandlung und/oder Verhinderung von Schrunden und/oder Flechten oder Pityriasis versicolor und/oder Rissen und/oder atopischer Dermatitis und/oder Ichtyosis und/oder Trockenheitszuständen der Haut oder von Schleimhäuten, die mit Haut- und/oder Schleimhautpathologien einhergehen, wie Ekzem, und/oder bei der Aufrechterhaltung oder der Verstärkung des Hydratationszustands von gereizter, empfindlicher, spröder, sensibilisierter, beeinträchtigter, überempfindlicher oder irritierter Haut und/oder gereizten, empfindlichen, spröden, sensibilisierten, beeinträchtigten, überempfindlichen oder irritierten Schleimhäuten und/oder bei der Behandlung von mit trockener Haut und/oder trockenen Schleimhäuten zusammenhängenden schuppigen Zuständen und/oder Juckreizen.

22. Pflegezusammensetzung, insbesondere Waschzusammensetzung, umfassend die Verbindung nach einem der Ansprüche 1 bis 8.

## Claims

1. Combination of pullulan or of an esterified derivative thereof or an organomineral silicon-based derivative thereof with a mixture of polysaccharides consisting of hyaluronic acid or a salt thereof or an esterified derivative thereof or an organomineral silicon-based derivative thereof and alginic acid or a salt thereof or an esterified derivative thereof or an organomineral silicon-based derivative thereof, the weight ratio of pullulan or esterified derivatives or organomineral silicon-based derivatives /hyaluronic acid, salts or esterified derivatives or organomineral silicon-based derivatives /alginic acid, salts or esterified derivatives or organomineral silicon-based derivatives being in the range 1/1/1 to 1/10/10.

2. Combination according to Claim 1, **characterized in that** the pullulan has a molecular weight of less than 500 kDa.

3. Combination according to either one of Claims 1 and 2, **characterized in that** the hyaluronic acid, the salts or esterified derivatives thereof have a molecular weight of greater than 20 kDa, advantageously between 50 and 800 kDa.

4. Combination according to any one of Claims 1 to 3, **characterized in that** it comprises a salt of hyaluronic acid, advantageously sodium hyaluronate.

5. Combination according to any one of Claims 1 to 4, **characterized in that** it comprises a salt of alginic acid, advantageously sodium alginate.

6. Combination according to any one of Claims 1 to 5 **characterized in that** the weight ratio of pullulan or esterified derivatives or organomineral silicon-based derivatives / hyaluronic acid, salts or esterified derivatives or organomineral silicon-based derivatives / alginic acid, salts or esterified derivatives or organomineral silicon-based derivatives is in the range 1/1/1 to 1/2/5.

7. Combination according to any one of Claims 1 to 6, **characterized in that** the weight ratio of pullulan, or esterified derivatives or organomineral silicon-based derivatives / alginic acid, salts or esterified derivatives or organomineral silicon-based derivatives is in the range 1/1 to 1/2.

8. Combination according to any one of Claims 1 to 7, **characterized in that** the weight ratio of pullulan or esterified derivatives or organomineral silicon-based derivatives / hyaluronic acid, salts or esterified derivatives or organomineral silicon-based derivatives / alginic acid, salts or esterified derivatives or organomineral silicon-based derivatives is 1/1/2.

9. Use of the combination according to any one of claims 1 to 8, for reducing the insensible water losses and/or for increasing the water content of the skin and/or mucous membranes and/or for increasing and/or prolonging the content of cosmetic and/or pharmaceutical active ingredients in the skin and/or the mucous membranes and/or maintaining it over time and/or as a moisturizing agent for the skin and/or the mucous membranes.

10. Mixture of cosmetic or pharmaceutical ingredients intended to be incorporated into a cosmetic or pharmaceutical composition, said mixture comprising the combination according to any one of Claims 1 to 8 in a content of between 0.1% and 5% by weight of dry matters relative to the total weight of the mixture of ingredients, advantageously between 1 and 3% by weight and a suitable cosmetic or pharmaceutical carrier.

11. Mixture of cosmetic or pharmaceutical ingredients according to Claim 10, **characterized in that** it comprises:
- pullulan or a cosmetically or pharmaceutically acceptable derivative thereof in a content between 0.01% and 3% by weight of dry matters relative to the total weight of the mixture of cosmetic or pharmaceutical ingredients, advantageously between 0.1% and 1% by weight; and
- hyaluronic acid or a cosmetically or pharmaceutically acceptable salt or derivative thereof in a content between 0.01% and 3% by weight of dry matters relative to the total weight of dry matters of the mixture of cosmetic or pharmaceutical ingredients, advantageously between 0.1% and 1% by weight; and/or
- alginic acid or a cosmetically or pharmaceutically acceptable salt or derivative thereof in a content between 0.01% and 3% by weight of dry matters relative to the total weight of the mixture of cosmetic or pharmaceutical ingredients.

12. Mixture of cosmetic or pharmaceutical ingredients according to any one of Claims 10 to 11, **characterized in that** it additionally comprises another moisturizing agent, in particular chosen from trehalose, serine, urea and mixtures thereof.

13. Mixture of cosmetic or pharmaceutical ingredients according to any one of Claims 10 to 12, **characterized in that** it contains alginic acid or a cosmetically or pharmaceutically acceptable salt or derivative thereof in a content between 0.1% and 2% by weight of dry matters relative to the total weight of the mixture of cosmetic or pharmaceutical ingredients.

14. Cosmetic or pharmaceutical composition intended for topical administration comprising the combination according to any one of Claims 1 to 8 in a content between 0.0001% and 20% by weight of dry matters relative to the total weight of the composition, advantageously between 0.01% and 5% by weight, and a suitable cosmetic or pharmaceutical carrier.

15. Composition according to Claim 14, **characterized in that** it comprises:
- pullulan or a cosmetically or pharmaceutically acceptable esterified derivative thereof or a cosmetically or pharmaceutically acceptable organomineral silicon-based derivative thereof in a content between 0.0001% and 10% by weight of dry matters relative to the total weight of the composition, advantageously between 0.005% and 0.1% by weight; and
- hyaluronic acid or a cosmetically or pharmaceutically acceptable salt thereof or a cosmetically or pharmaceutically acceptable esterified derivative thereof or a cosmetically or pharmaceutically acceptable organomineral silicon-based derivative thereof in a content between 0.0001% and 10% by weight of dry matters relative to the total weight of dry matters of the composition, advantageously between 0.005% and 0.1% by weight; and/or
- alginic acid or a cosmetically or pharmaceutically acceptable salt thereof or a cosmetically or pharmaceutically acceptable esterified derivative thereof or a cosmetically or pharmaceutically acceptable organomineral silicon-based derivative thereof in a content between 0.0001% and 10% by weight of dry matters relative to the total weight of the composition, advantageously between 0.007% and 0.2% by weight.

16. Composition according to any one of Claims 14 or 15, **characterized in that** it contains alginic acid or a cosmetically or pharmaceutically acceptable salt thereof or a cosmetically or pharmaceutically acceptable esterified derivative thereof or a cosmetically or pharmaceutically acceptable organomineral silicon-based derivative thereof in a content between 0.001% and 1% by weight of dry matters relative to the total weight of the composition.

17. Composition according to Claim 16, **characterized in that** it contains alginic acid or a cosmetically or pharmaceutically acceptable salt thereof or a cosmetically or pharmaceutically acceptable esterified derivative thereof or a cosmetically or pharmaceutically acceptable organomineral silicon-based derivative thereof in a content between 0.007% and 0.2% by weight of dry matters relative to the total weight of the composition.

18. Composition according to any one of Claims 14 to 17, **characterized in that** it is in the form of a serum, a lotion, a cream, a milk, an ointment, a paste, a foam, an emulsion, a hydrogel, a shower gel, a mask, a stick, a patch, or makeup powders, advantageously a cream or a lotion.

19. Composition according to any one of Claims 14 to 18, **characterized in that** it contains the combination of pullulan, or esterified derivatives or organomineral silicon-based derivatives / hyaluronic acid, salts or esterified derivatives or organomineral silicon-based derivatives / alginic acid, salts or esterified derivatives or organomineral silicon-based derivatives in a ratio in the range 1/1/1 to 1/2/5.

20. Use of the cosmetic composition according to any one of Claims 14 to 19, for maintaining or improving the state of hydration of the skin and/or mucous membranes, in particular of senile skin and/or mucous membranes, and/or for preventing or slowing down the appearance of the signs of cutaneous and/or mucosal dryness, in particular over at least 10 hours, and/or for treating cutaneous and/or mucosal dryness conditions which are the tautness associated with dry skin and/or mucous membranes and/or for preventing or reducing the appearance of wrinkles linked to cutaneous dryness, and/or for improving the comfort of dry skin and/or mucous membranes, and/or for treating skin and/or mucous membranes having an appearance that is rough to look at and/or to the touch.

21. Pharmaceutical composition according to any one of Claims 14 to 19, for the use thereof in the treatment and/or the prevention of fissures and/or scurf or pityriasis alba and/or cracks and/or atopic dermatitis and/or ichthyosis and/or conditions of dryness of the skin or mucous membrane that accompany cutaneous and/or mucosal pathologies such as eczema, and/or for maintaining or improving the state of hydration of the skin and/or mucous membranes of aggressed, sensitive, sensitized, impaired, intolerant, fragile or reactive skin and/or mucous membranes, and/or in the treatment of the squamous states and/or itching associated with dry skin and/or mucous membranes.

22. Cleansing composition, in particular detergent composition, comprising the combination according to any one of Claims 1 to 8.
